# EUROPEAN PATENT APPLICATION

(11) **EP 2 997 950 A2**
(43) Date of publication of application: **23.03.2016**
(21) Application number: 14797637.7
(22) Date of filing: 14.05.2014
(51) Int. Cl.: A61G 9/00, A61F 5/44

(54) **AUTOMATIC URINE COLLECTION DEVICE**

(30) Priority: 15.05.2013 KR 20130055368; 13.05.2014 KR 20140057328
(71) Applicant: Hanmedics Co, Ltd., Seoul 136-075 (KR); Kim, Kyoung-Hun, Dongdaemun-gu, Seoul 130-719 (KR)
(72) Inventor: KIM, Kyoung-Hun, Seoul 130-719 (KR)
(74) Representative: Merryweather, Colin Henry
(86) International application number: PCT/KR2014/004339
(87) International publication number: WO 2014/185715

(57) **Abstract**

The present invention relates to an automatic urine collection apparatus, which comprises: a urine collecting unit 10 for collecting urine from a user and spraying a cleansing water to a urination part; a housing 20 comprising a urine storage unit 21 for storing the urine collected from the urine collecting unit 10, a cleansing water storage unit 22 for storing the cleansing water used in the urine collecting unit 10, a driving unit 23 for moving the urine in the urine collecting unit 10 to the urine storage unit 21 and moving the cleansing water in the cleansing water storage unit 22 to the urine collecting unit 10, a control unit 24 for controlling an operation of the driving unit 23, a filtration unit 25, and a housing sensor unit including sensors for detecting a urine level of the urine storage unit 21, a cleansing water level of the cleansing water storage unit 22, and a temperature of the cleansing water; and an operational panel 30 for manipulating a control and checking a state of the control unit 24. The present apparatus allows a patient to urinate by himself/herself without help of a caregiver, allows a bedridden patient who is unable to wash the urination part by himself/herself to wash the urination part with the cleansing water after urination for sanitary purpose, relieves the mental and economic burdens by minimizing urinary tract infections, and prevents an offensive odor during use by equipping a deodorizing means for removing the offensive odor released through the air outlet of the urine storage unit in which the urine is stored.

## Description

### [TECHNICAL FIELD]

The present invention relates to an urine collection apparatus for easily collecting urine of elderly or weak persons or patients. More particularly, the present invention relates to an automatic urine collection apparatus, which allows elderly or weak persons or patients themselves to comfortably urinate without help of a caregiver and to thoroughly wash their urination part with a cleaning water after urination, thereby maintaining the sanitary condition of bedridden patients or elderly or weak persons.

### [BACKGROUND ART]

Patients who are unable to use bathrooms for urination, for example, most patients with serious illness, patients who cannot move their lower parts of bodies, bedridden patients with chronic degenerative diseases, or elderly or weak persons with urinary incontinence, must urinate in the bed with the help of a caregiver. When a bedridden patient urinates, at least two caregivers are needed; one lifts the patient's pelvis and another locates a urinal receptacle under the buttocks of the patient.

However, such a approach makes both the patient and caregivers feel inconvenient and may bring an adverse effect on treatment for the patient who needs to restrict the movement and rest. To avoid these problems, foley catheter insertion operations into the urethra are often conducted even for the patient having urination ability.

This operation is to insert a foley catheter from the entrance of urethra to the bladder; the patient suffers from an intolerable pain and female patients may feel shameful during the operation. More serious problems include infections of urinary system. In fact, the most common reason for the post-operative infection is urinary tract infection(UTI). It is difficult to maintain the foley catheter in the body for a long time (more than 7 days) due to such infections of urinary system such as urethra and bladder. Actually, when the patient shows a symptom of infection such as rigor and fever, the first step in the hospital is to remove the foley catheter, administer an appropriate antibiotics to a patient, and then watch its course. Once infection is diagnosed, the foley catheter should be removed, and conservative treatment concurrent with the administration of various antibiotics is performed, which prolongs the hospitalization period inevitably. This may cause excessive economic loss and mental burden for the patient. Despite these problems, foley catheter operation has been selectively performed for the patients who have urination ability but have inconvenient behavior

As one example of conventional urine collection apparatus developed in consideration of these circumstances, Korean Patent Nos. 0367231 and 0374235 disclose a personal urine collection and bidet apparatus.

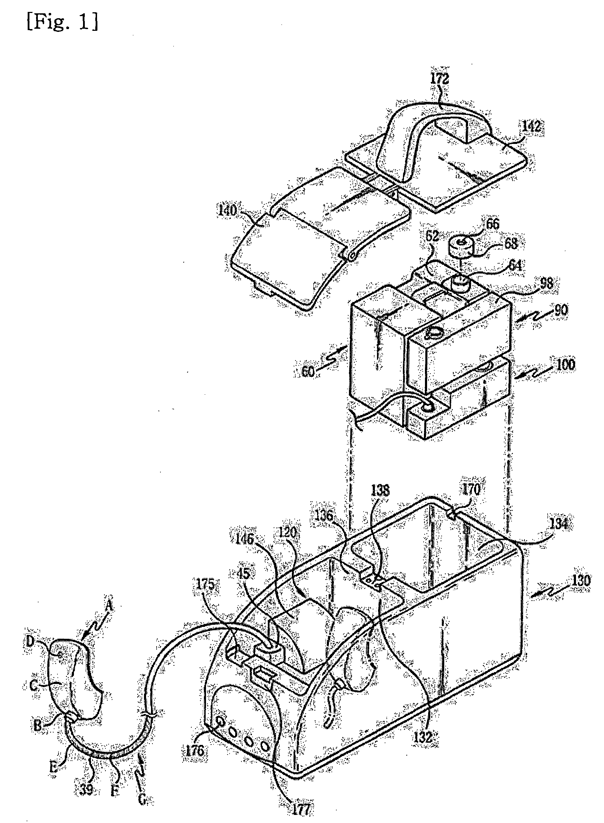

The personal urine collection and bidet apparatus of Patent Nos. 0367231 and 0374235 comprises, as shown in Fig. 1, a urine collecting unit A for collecting urine from a user and spraying a cleansing water to a urination part, which includes a urine collecting device C for collecting the spouted urine and draining it through an outlet B formed on the bottom surface with a ventilation means for ventilating outside air to the urination part of the user, a bidet D installed on one side of the urine collecting device C for spraying the cleansing water to the urination part, a connecting pipe G having a Foley catheter E installed to be connected to the outlet B to drain the urine, a cleansing water pipe F for delivering the cleansing water, and an electric wire 39 included therein, and a coupling member 45 for coupling to the connecting pipe; a urine storage unit 60 for storing the urine collected in the urine collecting unit A, which includes a urine storage tank 62 having a space formed to store a prescribed liquid therein, an inlet 64 formed on one side of the urine storage tank 62 to flow the liquid, and a cap 68 detachably coupled to the inlet 64 and having a through hole 66 in the center thereof; a cleansing water storage unit 90 for storing the cleansing water used in the urine collecting unit 10, which includes a cleansing water storage tank 98 in which a space is formed therein to contain the cleansing water therein, a cleansing water supply hole is formed to fill the cleansing water in an upper part, and a cleansing water discharge hole is formed on a lower part for discharging the cleansing water via a check valve for controlling the flow of the cleansing water, and a heating unit 100 for heating the cleansing water supplied through the cleansing water discharge hole at a prescribed temperature and delivering it to the urine collecting unit; a driving unit 120 for moving the urine in the urine collecting unit A to the urine storage unit 60 and moving the cleansing water in the cleansing water storage unit 90 to the urine collecting unit A; a control unit for controlling operations of the driving unit 120; and a housing 130 for accommodating the urine storage unit 60, the cleansing water storage unit 90, the driving unit 120, and the control unit within, which includes a front reception unit 132 for installing the urine collecting unit 10 detachably, a rear reception unit 134 for installing the urine storage unit 60 and the cleansing water storage unit 90, a partition for separating the front and rear reception units 132 and 134, a hinge coupling unit 138 installed on an upper central portion of the partition 136, front and rear covers 140 and 142 having handles 172 located on upper parts of the front and rear reception units 132 and 134 and installed rotatable using the hinge coupling unit 138 as a shaft, respectively, a driving unit housing 146, a rear discharge groove 170 formed on a rear end portion, a front discharge groove 175 formed on a front end portion, and an operational switch and open button 177 placed on a front surface, which allows a patient, an elderly person, or a weak person to urinate conveniently.

However, the above personal urine collection and bidet apparatus disclosed in Patent Nos. 0367231 and 0374235 has a drawback of giving out an offensive odor during use because it is not equipped with any deodorizing means for removing the offensive odor discharged from the air outlet of the urine storage unit 60 of the housing 130.

Moreover, since the cleansing water storage unit 90 is provided with narrow openings of entrance and exit and contaminated by forming water scale or the like in the cleansing water storage unit 90, there was a problem in that their washing is not possible.

In addition, both the urine storage tank 62 of the urine storage unit 60 and the cleansing water storage tank 98 of the cleansing water storage unit 90 are accommodated in the rear reception unit 134 together and the inlet 64 of the urine storage tank 62 and the inlet of the cleansing water storage tank 98 are adjacent to each other in the same direction, which causes the problem of contamination of the cleansing water in the cleansing water storage tank 98 with the urine in the urine storage tank 62.

### [DISCLOSURE OF INVENTION]

### [TECHNICAL PROBLEM]

The present invention has been made keeping in mind the above drawbacks and problems encountered with the personal urine collecting and bidet apparatus described above, and an object of the present invention is to provide an automatic urine collection apparatus configured such that bedridden patients who are unable to use the toilet can conduct urination without help of a caregiver; even for patients who cannot freely move their arms, a caregiver can help urination of the patients with ease; the patients themselves can wash the urination part with a cleansing water after urination, thereby maintaining the sanitary condition of the bedridden patients who are unable to wash the urination part without help of a caregiver; and urinary tract infection can be minimized, thereby reducing the mental and economic burdens of the patients.

Another object of the present invention is to provide an automatic urine collecting apparatus configured such that a sanitary condition of patients can be maintained by supplying air to the urination part after its washing and then drying the urination part of the patient which is wet with the cleansing water.

Yet another object of the present invention is to provide an automatic urine collection apparatus configured so as to include a deodorizing means for removing the offensive odor released through the air outlet in the urine storage unit where the urine is stored in the urine storage unit of the housing, thereby preventing an offensive odor during its usage.

Yet another object of the present invention is to provide an automatic urine collection apparatus configured such that the housings where the urine storage tank of the urine storage unit is accommodated and where the cleansing water storage tank of the cleansing water storage unit is accommodated are separated and also the opening for receiving the urine storage tank and the opening for receiving the cleansing water storage tank are directed to lower left side and upper right side, respectively, which is not unidirectional, thereby fundamentally eliminating the possibility of contamination of the cleansing water with the urine in the urine storage tank and facilitating washing of both the urine storage tank and the cleansing water storage tank.

Yet another object of the present invention is to provide an automatic urine collection apparatus which is capable of operating the pump by detecting a gathering of any liquid including cleansing water, an antiseptic solution or the like as well as watery feces in the urine gathering device, which improves the reliability of the product.

Yet another object of the present invention is to provide an automatic urine collection apparatus configured so as to be in close contact with the urine gathering device gently around the urination part, thereby water tightness is increased and at the same time the user has a sense of stability to use it comfortably.

Yet another object of the present invention is to provide an automatic urine collection apparatus configured so as to make the total conduit of the urine gathering device detachable from the housing, thereby facilitating cleaning and replacement.

Yet another object of the present invention is to provide an automatic urine collection apparatus configured such that the cleansing water is supplied at a proper temperature while washing the urination part of the patient after urination, thereby the patient can comfortably wash the urination part without a burden and also the means for shutting down the power supply is provided to prevent the cleansing water from overheating above a certain temperature, thereby the patient can use it safely without the concern of the safety accidents.

### [TECHNICAL SOLUTION]

To achieve the above objects, the present invention provides an automatic urine collection apparatus which comprises: a urine collecting unit 10 for collecting urine from a user and spraying a cleansing water to a urination part; a housing 20 comprising a urine storage unit 21 for storing the urine collected from the urine collecting unit 10, a cleansing water storage unit 22 for storing the cleansing water used in the urine collecting unit 10, a driving unit 23 for moving the urine in the urine collecting unit 10 to the urine storage unit 21 and moving the cleansing water in the cleansing water storage unit 22 to the urine collecting unit 10, a control unit 24 for controlling an operation of the driving unit 23, a filtration unit 25, and a housing sensor unit including sensors for detecting a urine level of the urine storage unit 21, a cleansing water level of the cleansing water storage unit 22, and a temperature of the cleansing water; and an operational panel 30 for manipulating a control of the control unit 24 and checking its state.

### [ADVANTAGEOUS EFFECTS]

The automatic urine collection apparatus according to the present invention has the following advantageous effects. Bedridden patients who are unable to use the toilet can conduct urination without help of a caregiver; even for patients who cannot freely move their arms, a caregiver can help urination of the patients with ease; the patients themselves can wash the urination part with a cleansing water after urination, thereby maintaining the sanitary condition of the bedridden patients who are unable to wash the urination part without help of a caregiver; and urinary tract infection can be minimized, thereby reducing the mental and economic burdens of the patients. Moreover, a sanitary condition of patients can be maintained by supplying air to the urination part after its washing and then drying the urination part of the patient which is wet with the cleansing water. Further, a deodorizing means for removing the offensive odor released through the air outlet in the urine storage unit where the urine is stored in the urine storage unit of the housing, is provided, thereby preventing an offensive odor during its usage. In addition, the housings where the urine storage tank of the urine storage unit is accommodated and where the cleansing water storage tank of the cleansing water storage unit is accommodated are separated and also the opening for receiving the urine storage tank and the opening for receiving the cleansing water storage tank are directed to lower left side and upper right side, respectively, which is not unidirectional, thereby fundamentally eliminating the possibility of contamination of the cleansing water with the urine in the urine storage tank and facilitating washing of both the urine storage tank and the cleansing water storage tank.

### [BRIEF DESCRIPTION OF THE DRAWINGS]

The above and other objects, features and advantages of the present invention will be more clearly understood from the following detailed description taken in conjunction with the accompanying drawings, in which:
Fig. 1 is an exploded perspective view showing a configuration of a conventional personal urine collection and bidet apparatus,
Fig. 2 is a block diagram showing a configuration of an automatic urine collection apparatus of the present invention,
Fig. 3 is a perspective view showing a configuration of an automatic urine collection apparatus of the present invention,
Fig. 4a is an exploded perspective view of a housing according to the present invention,
Fig. 4b illustrates a body of a urine storage tank and a coupling means of a cap portion according to the present invention,
Fig. 5a is a perspective view of a urine collecting cup for women to which a urine gathering unit is coupled according to an embodiment of the present invention,
Fig. 5b is a perspective view of a urine collecting cup for women in which a urine gathering unit is separated according to an embodiment of the present invention,
Fig. 5c is an exploded perspective view of a urine collecting cup for women of silicon replacement type in which the nose portion of the silicon is low according to an embodiment of the present invention,
Fig. 5d is an exploded perspective view showing a silicon bottom surface of a urine collecting cup for women of silicon replacement type in which the nose portion of the silicon is low according to an embodiment of the present invention,
Fig. 5e is an exploded perspective view of a urine collecting cup for women of silicon replacement type in which the nose portion of the silicon is high according to an embodiment of the present invention,
Fig. 5f is an exemplary view showing a state where a coupling protrusion is formed on a support of a cup body and a coupling hole is formed on a bottom surface of a silicon according to an embodiment of the present invention,
Fig. 5g is an exemplary view showing a state where a plurality of wrinkle bands are formed on front surfaces of left and right sides of a cup body and a plurality of wrinkle strips are formed around a silicon according to an embodiment of the present invention,
Fig. 6a is an exemplary view of a silicon of a urine collecting cup for women of silicon replacement type in which the nose portion of the silicon is low according to the present invention,
Fig. 6b is an exemplary view of a silicon of a urine collecting cup for women of silicon replacement type in which the nose portion of the silicon is high according to the present invention,
Fig. 7a is a perspective view of a urine collecting cup for men to which a urine gathering unit is coupled according to the present invention,
Fig. 7b is an exploded perspective view of a urine collecting cup for men according to the present invention,
Fig. 7c is an exploded perspective view showing an embodiment of a urine collecting cup for men according to the present invention,
Fig. 7d is an exploded perspective view showing an embodiment of a urine collecting cup for men according to the present invention,
Fig. 8 is an exploded perspective view of an adaptor according to the present invention,
Figs. 9a to 9f are exploded perspective views of urine storage tanks according to an embodiment of the present invention,
Fig. 10 is an exemplary view of an automatic urine collecting apparatus of the present invention configured to be wall mountable,
Fig. 11 is an exploded perspective view illustrating an installation state of a circular plate type heater according to the present invention,
Fig. 12a is a partly cutout exploded perspective view of a deodorizing filter according to the present invention,
Fig. 12b is a sectional view of a deodorizing filter according to the present invention,
Fig. 13a shows a configuration of an embodiment of a filtration unit according to the present invention,
Fig. 13b shows a configuration of an embodiment of a filtration unit according to the present invention,
Fig. 14 illustrates a coupling state of an adaptor and a housing according to the present invention,
Fig. 15 is a perspective view of a wheelchair mounting means according to the present invention,
Fig. 16 illustrates a use state of a wheelchair mounting means according to the present invention,
Fig. 17 illustrates a use state of a urine collecting cup using a girdle according to the present invention,
Fig. 18a shows a configuration of an X girdle for women according to the present invention,
Fig. 18b illustrates a use state of a urine collecting cup using an X girdle for women according to the present invention,
Fig. 19a shows a configuration of an X girdle for men according to the present invention, and
Fig. 19b illustrates a use state of a urine collecting cup using X girdle for men according to the present invention.

### [BEST MODE CARRYING OUT THE INVENTION]

Now, preferred embodiments of the automatic urine collection apparatus of the present invention are described in detail with reference to accompanying drawings.

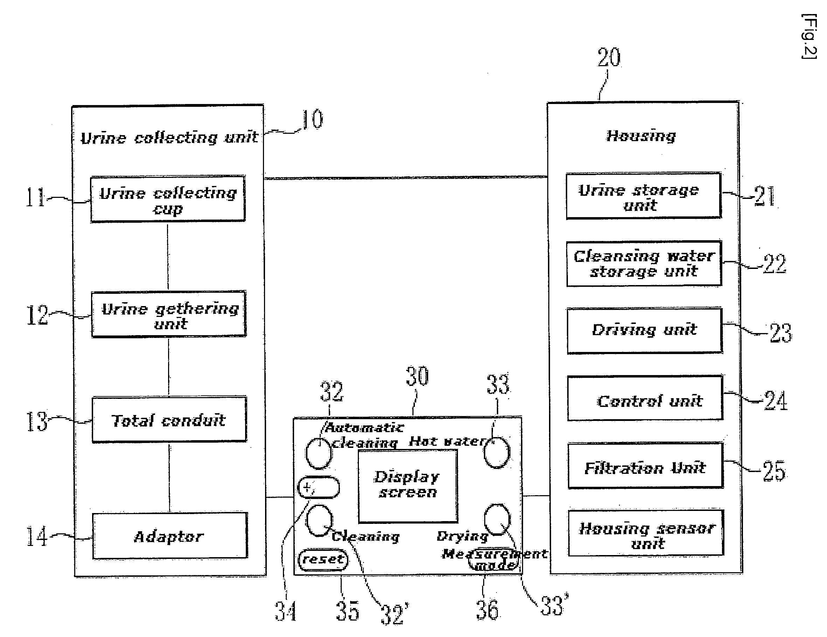

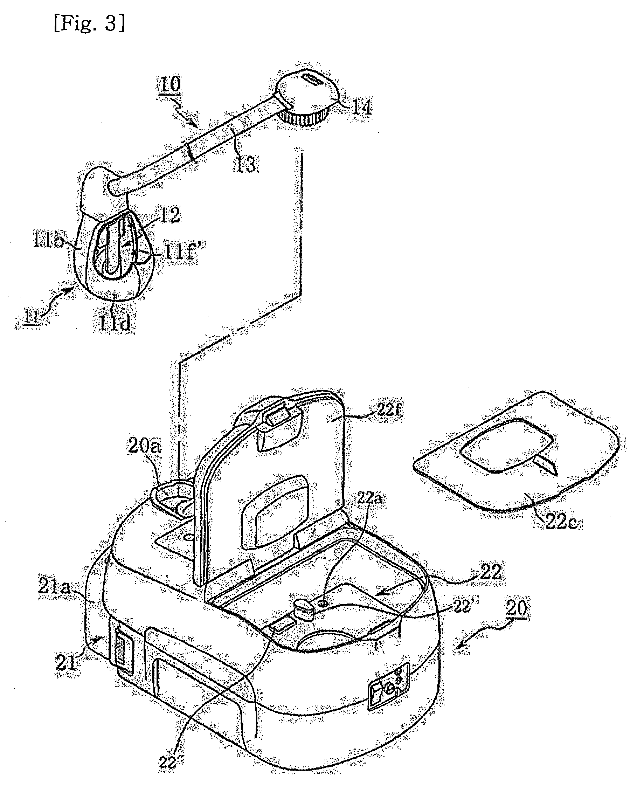

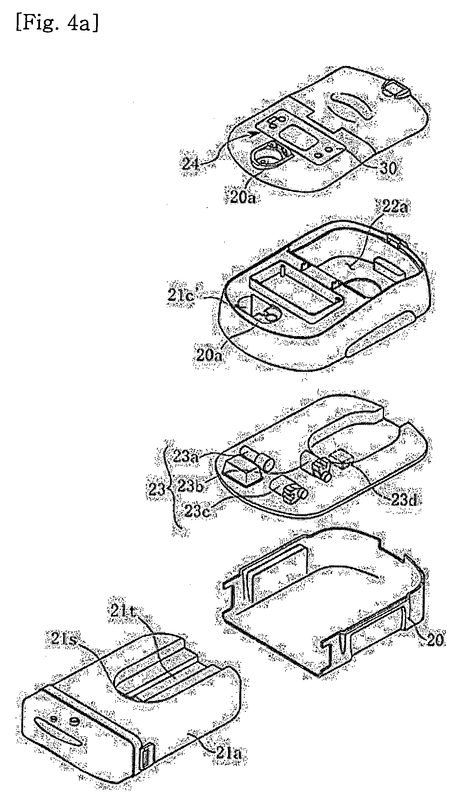

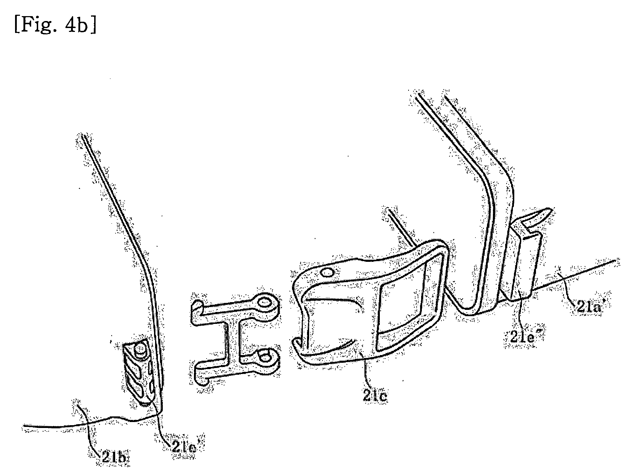

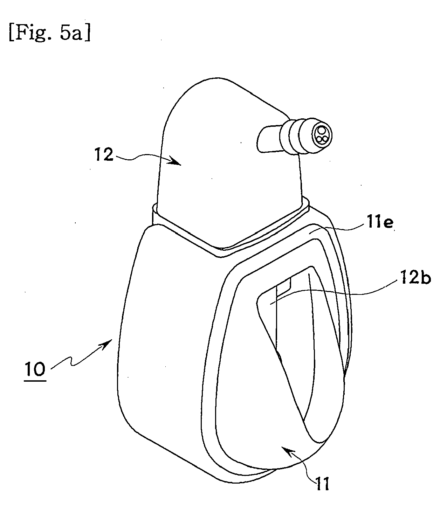

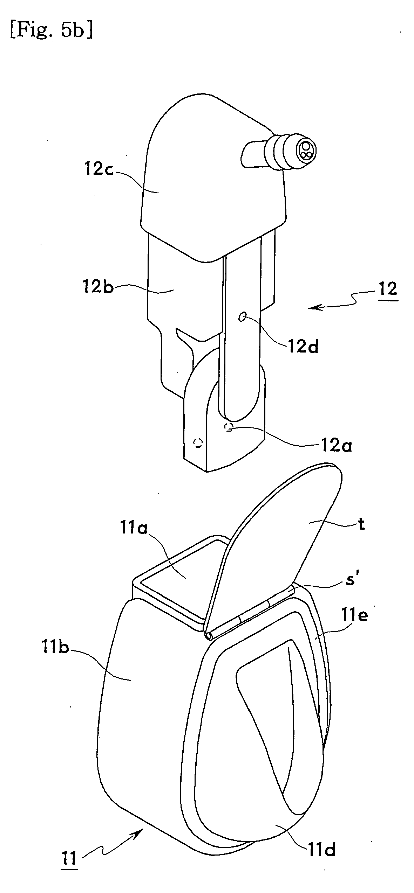

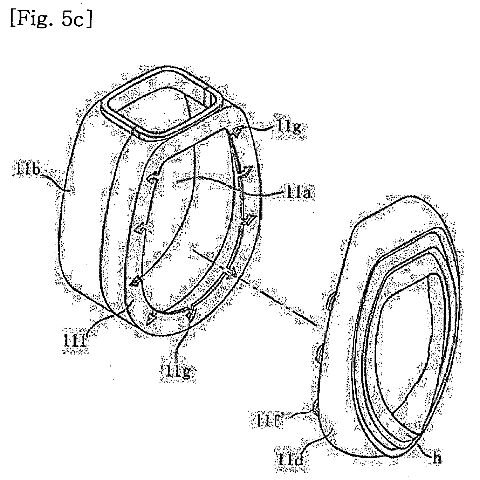

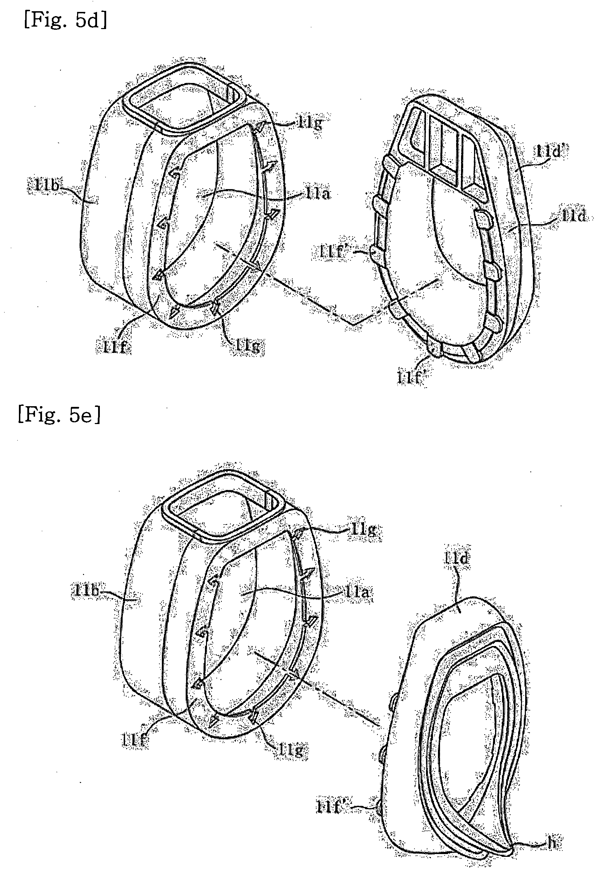

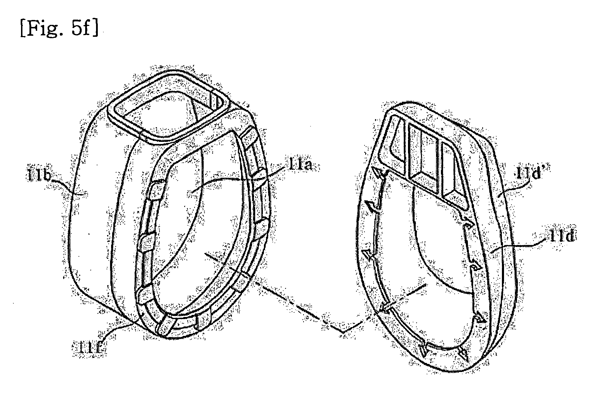

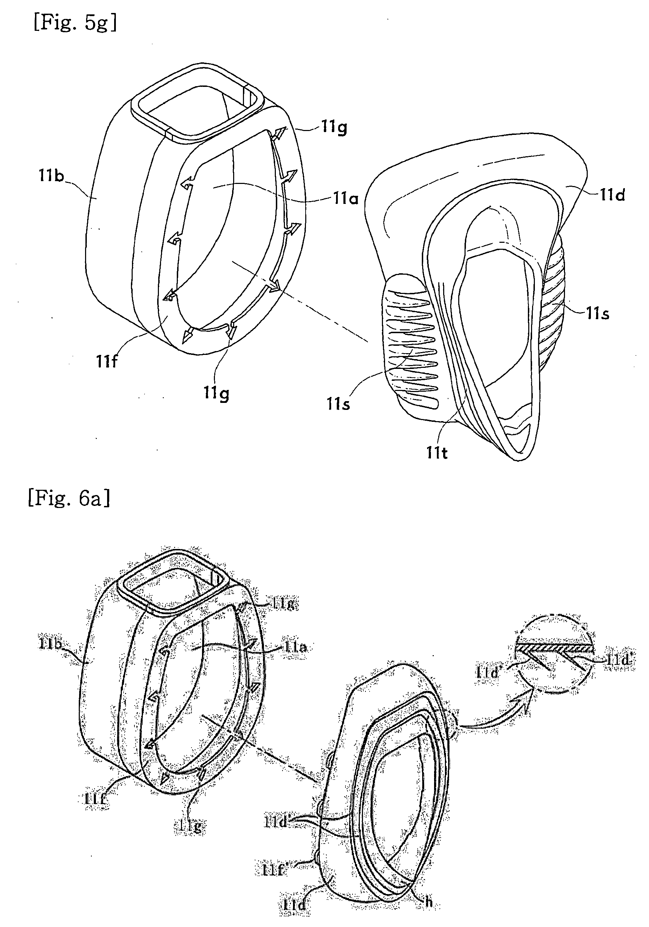

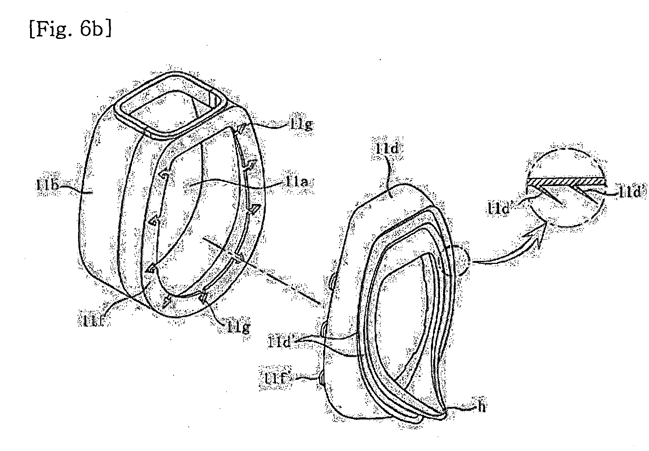

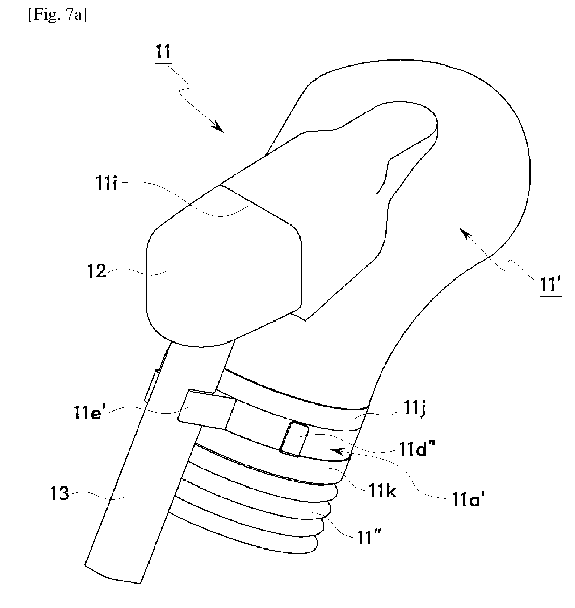

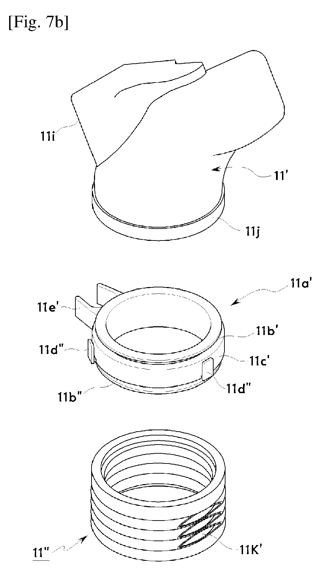

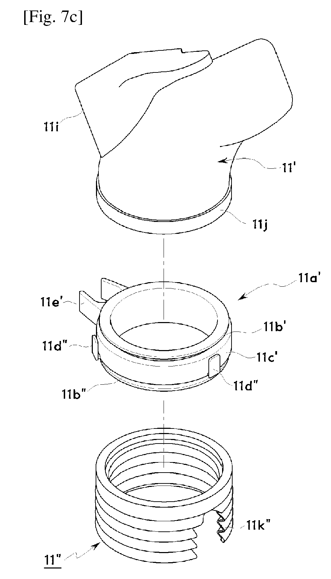

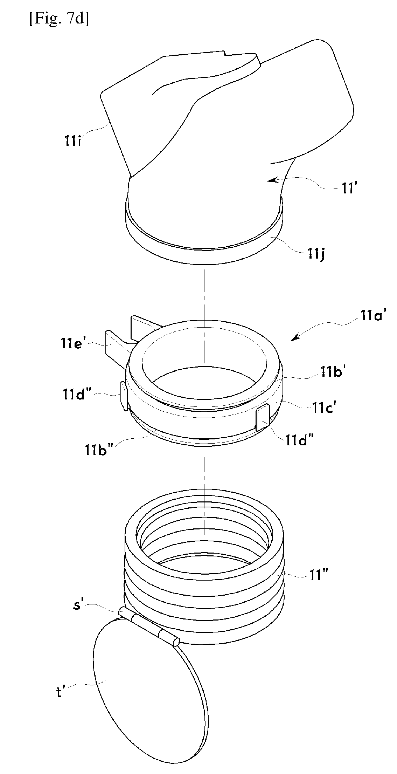

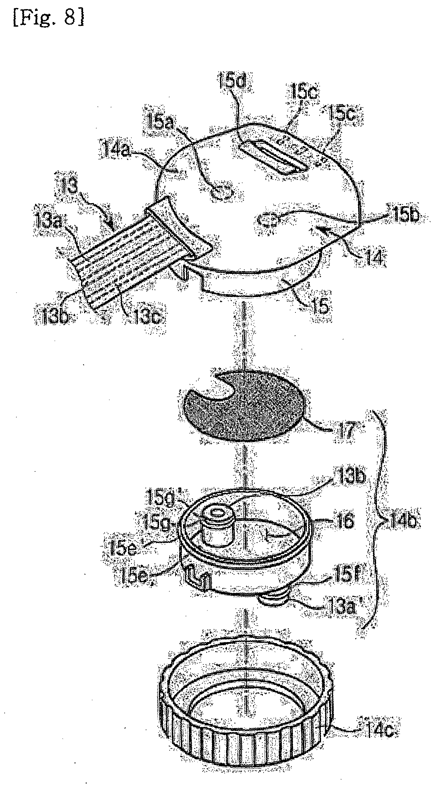

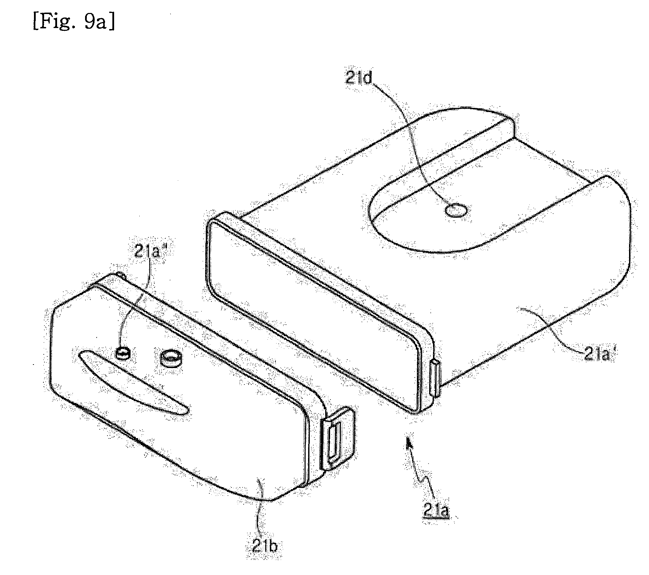

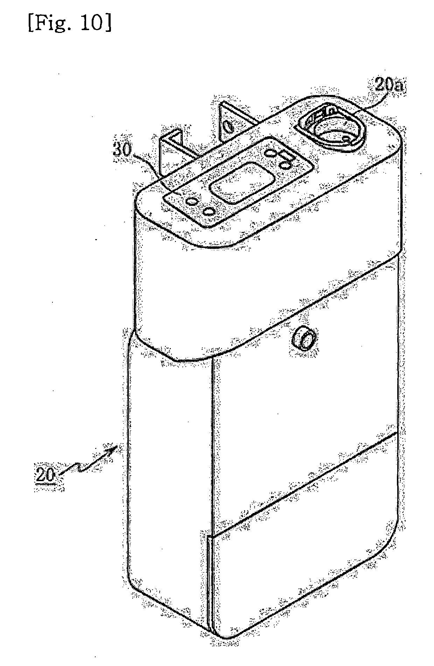

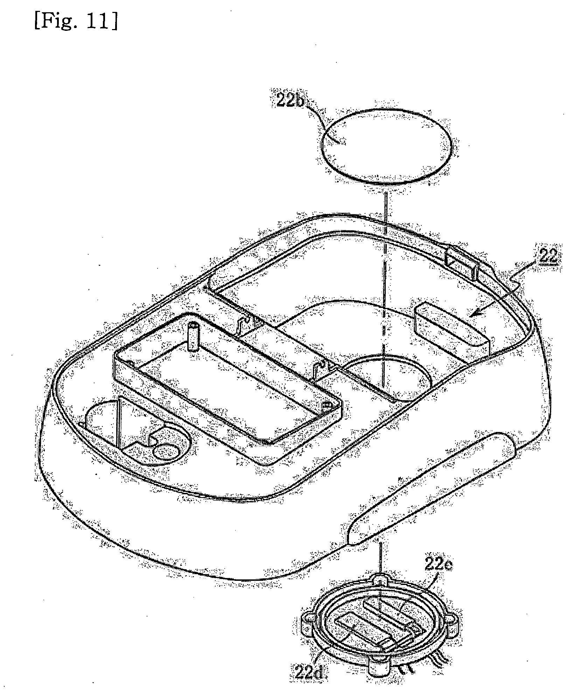

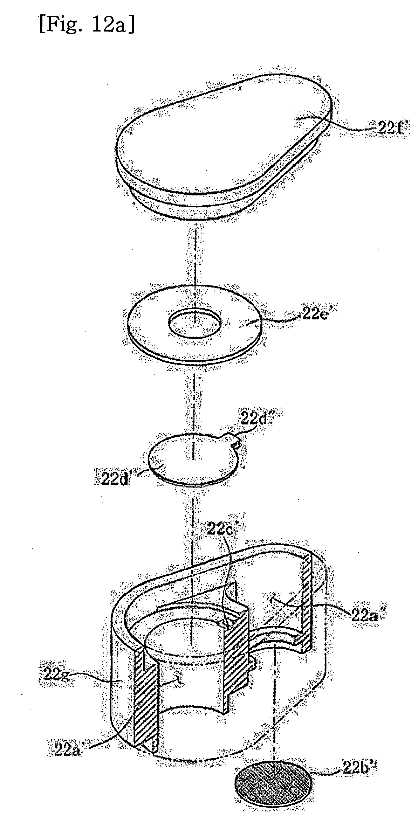

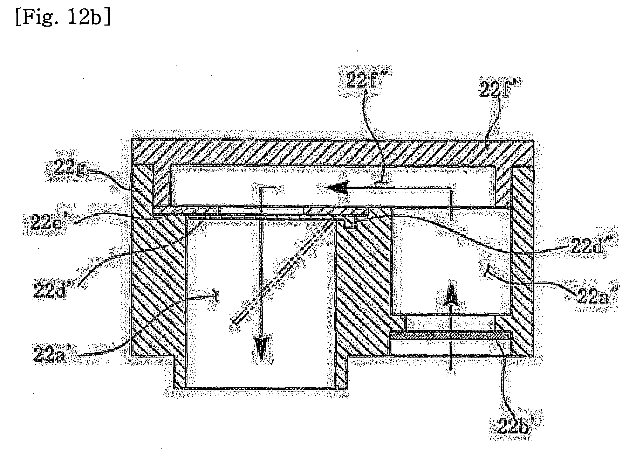

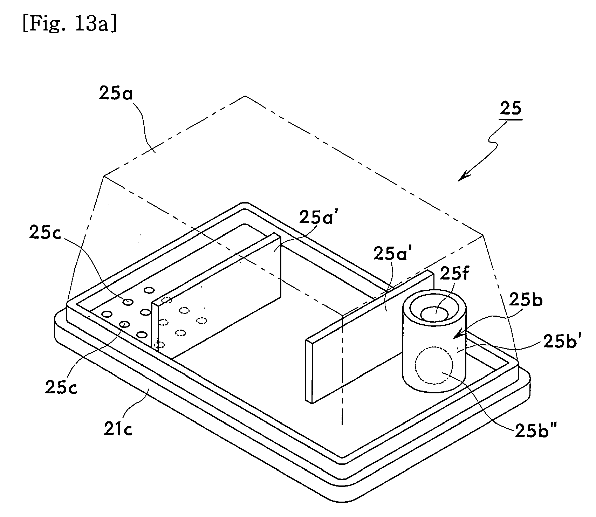

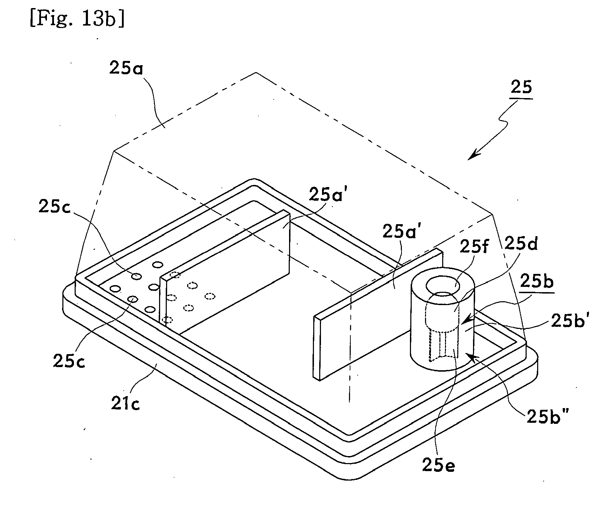

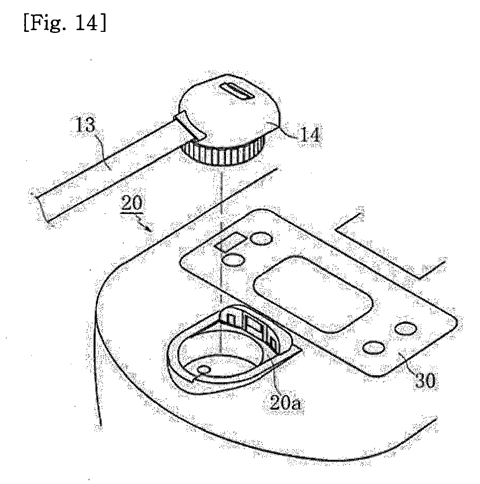

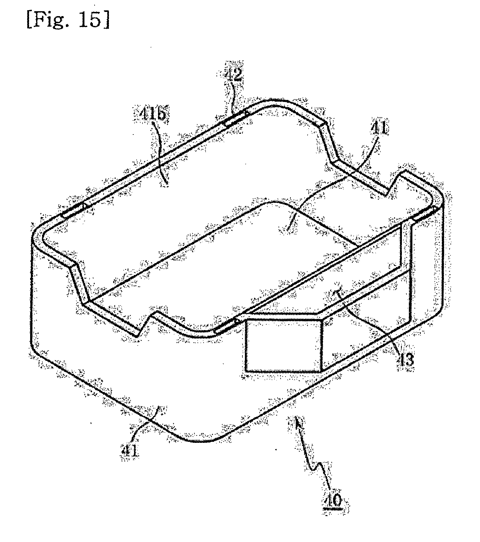

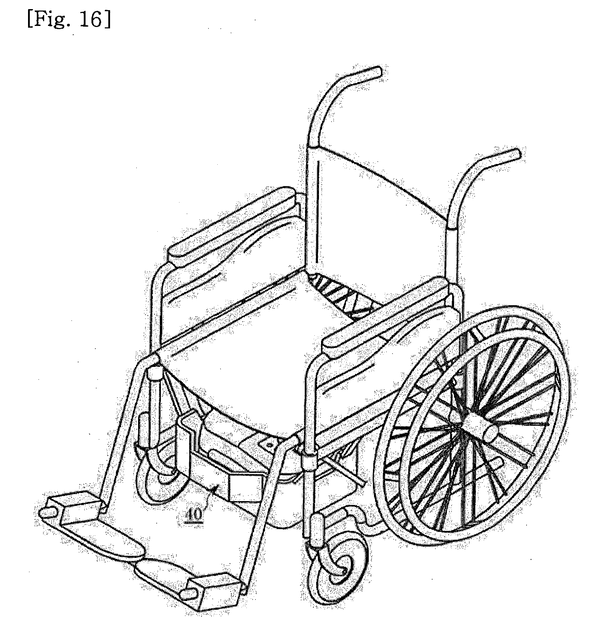

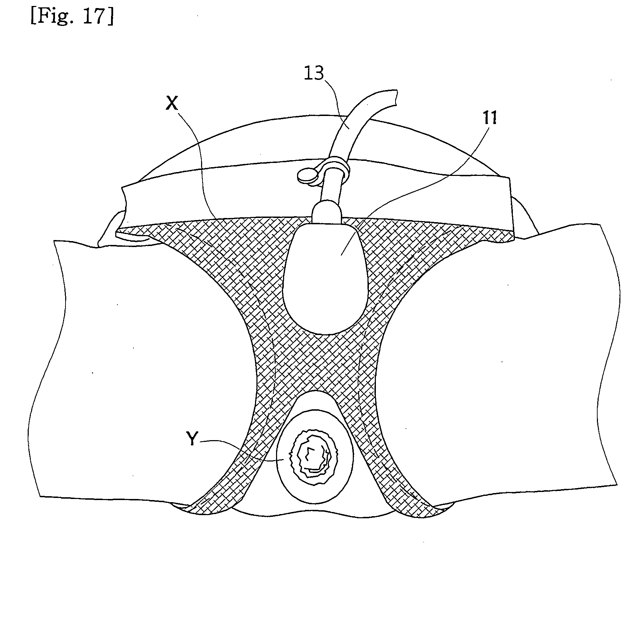

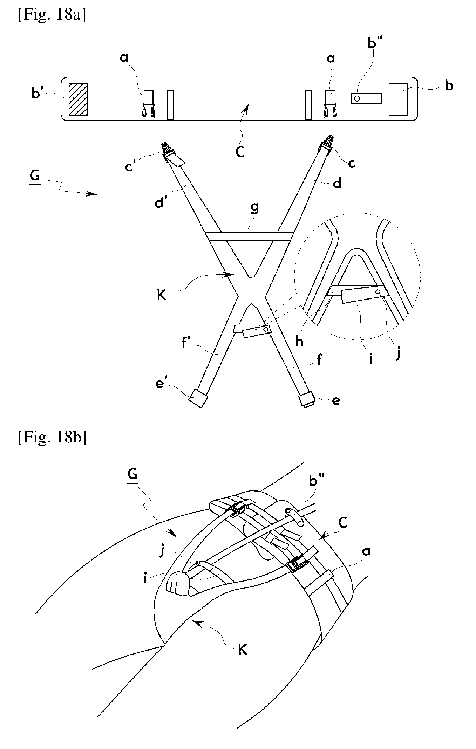

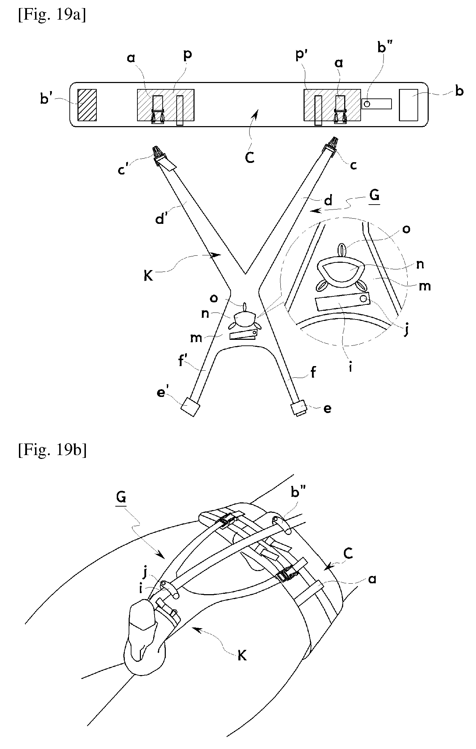

Fig. 2 is a block diagram showing a configuration of an automatic urine collection apparatus of the present invention, Fig. 3 is a perspective view showing a configuration of an automatic urine collection apparatus of the present invention, Fig. 4a is an exploded perspective view of a housing according to the present invention, Fig. 4b illustrates a body of a urine storage tank and a coupling means of a cap portion according to the present invention, Fig. 5a is a perspective view of a urine collecting cup for women to which a urine gathering unit is coupled according to an embodiment of the present invention, Fig. 5b is a perspective view of a urine collecting cup for women in which a urine gathering unit is separated according to an embodiment of the present invention, Fig. 5c is an exploded perspective view of a urine collecting cup for women of silicon replacement type in which the nose portion of the silicon is low according to an embodiment of the present invention, Fig. 5d is an exploded perspective view showing a silicon bottom surface of a urine collecting cup for women of silicon replacement type in which the nose portion of the silicon is low according to an embodiment of the present invention, Fig. 5e is an exploded perspective view of a urine collecting cup for women of silicon replacement type in which the nose portion of the silicon is high according to an embodiment of the present invention, Fig. 5f is an exemplary view showing a state where a coupling protrusion is formed on a support of a cup body and a coupling hole is formed on a bottom surface of a silicon according to an embodiment of the present invention, Fig. 5g is an exemplary view showing a state where a plurality of wrinkle bands are formed on front surfaces of left and right sides of a cup body and a plurality of wrinkle strips are formed around a silicon according to an embodiment of the present invention, Fig. 6a is an exemplary view of a silicon of a urine collecting cup for women of silicon replacement type in which the nose portion of the silicon is low according to the present invention, Fig. 6b is an exemplary view of a silicon of a urine collecting cup for women of silicon replacement type in which the nose portion of the silicon is high according to the present invention, Fig. 7a is a perspective view of a urine collecting cup for men to which a urine gathering unit is coupled according to the present invention, Fig. 7b is an exploded perspective view of a urine collecting cup for men according to the present invention, Fig. 7c is an exploded perspective view showing an embodiment of a urine collecting cup for men according to the present invention, Fig. 7d is an exploded perspective view showing an embodiment of a urine collecting cup for men according to the present invention, Fig. 8 is an exploded perspective view of an adaptor according to the present invention, Figs. 9a to 9f are exploded perspective views of urine storage tanks according to an embodiment of the present invention, Fig. 10 is an exemplary view of an automatic urine collecting apparatus of the present invention configured to be wall mountable, Fig. 11 is an exploded perspective view illustrating an installation state of a circular plate type heater according to the present invention, Fig. 12a is a partly cutout exploded perspective view of a deodorizing filter according to the present invention, Fig. 12b is a sectional view of a deodorizing filter according to the present invention, Fig. 13a shows a configuration of an embodiment of a filtration unit according to the present invention, Fig. 13b shows a configuration of an embodiment of a filtration unit according to the present invention, Fig. 14 illustrates a coupling state of an adaptor and a housing according to the present invention, Fig. 15 is a perspective view of a wheelchair mounting means according to the present invention, Fig. 16 illustrates a use state of a wheelchair mounting means according to the present invention, Fig. 17 illustrates a use state of a urine collecting cup using a girdle according to the present invention, Fig. 18a shows a configuration of an X girdle for women according to the present invention, Fig. 18b illustrates a use state of a urine collecting cup using an X girdle for women according to the present invention, Fig. 19a shows a configuration of an X girdle for men according to the present invention, Fig. 19b illustrates a use state of a urine collecting cup using X girdle for men according to the present invention. The automatic urine collection apparatus of the present invention comprises: a urine collecting unit 10 for collecting urine from a user and spraying a cleansing water to a urination part; a housing 20 comprising a urine storage unit 21 for storing the urine collected from the urine collecting unit 10, a cleansing water storage unit 22 for storing the cleansing water used in the urine collecting unit 10, a driving unit 23 for moving the urine in the urine collecting unit 10 to the urine storage unit 21 and moving the cleansing water in the cleansing water storage unit 22 to the urine collecting unit 10, a control unit 24 for controlling an operation of the driving unit 23, a filtration unit 25, and a housing sensor unit including sensors for detecting a urine level of the urine storage unit 21, a cleansing water level of the cleansing water storage unit 22, and a temperature of the cleansing water; and an operational panel 30 for manipulating a control of the control unit 24 and checking its state.

The urine collecting unit 10 comprises: a urine gathering unit 12 employed in the body side of the user; a total conduit 13 to which the urine gathering unit 12 and an adaptor 14 are connected; an adaptor 14 coupled to the housing.

The urine gathering unit 12 comprises: a urine gathering cervix part 12b comprising a structure for attaching or detaching the urine collecting cup 11 by being inserted into an opening 11a of an urine collecting cup 11 for gathering the spouted urine, and an opening 12d for supporting a sensor unit 12a to be rotatable and for spraying the cleansing water; and a urine gathering corpus part 12c connected to the total conduit 13.

The urine gathering cervix part 12b has a structure that is capable of attaching or detaching the urine collecting cup 11 by being inserted into the urine collecting cup 11 and allowing rotation of the urine sensor unit 12a. The urine gathering cervix part 12b also has an opening 12d for spraying the cleansing water and blowing dry air away. The urine gathering corpus part 12c is in one united body with the urine gathering cervix part 12b; however, it refers to a part which is protruded outside the urine collecting cup 11 when it is combined with the urine collecting cup 11, and it serves to connect to the total conduit 13.

The urine collecting cup 11 of the urine collecting unit 10 consists preferably of: a cup body 11b for securing a space in which the sensor unit 12a can rotate; and a silicon 11d or latex material which is employed in the user's body side and replaceable and which is harmless to humans.

Further, the urine collecting cup 11 is classified into for women and for men. The urine collecting cup 11 for women is configured such that a cup body 11b, which has an opening 11a for gathering urine on an upper part, a lower part of which has a semicircular shape, and a front surface of which is opened, to which a fixing means 11e of a ring shape is inserted and adhered; and a silicon 11d, a center portion of which is opened, is inserted and fused, thereby the cup body 11b, the silicon 11d and the fixing means 11e are integrally used for women.

It is preferable that an offensive odor is prevented by installing a cover t for covering the front open portion of the cup body 11b through a hinge s provided on an upper part of the fixing means 11e.

In the case of a silicon separation type, there is a coupling hole 11g in a support 11f of the cup body 11b of the urine collecting cup 11, and a coupling protrusion 11f' is formed on a bottom surface of the silicon 11d, the coupling direction of which can be classified into the one detachable in forward and backward directions and the other detachable laterally.

The coupling hole 11g and the coupling protrusion 11f' are preferably formed to have barb shapes, which are not to be easily missed after the coupling protrusion 11f' is inserted laterally. The silicon 11d may be diversified to the ones with high or low nose portion 11h (with long or short vertical distance from the bottom surface on which the protrusion 11f' is formed), thus improving a contact feeling to the female perineum. It is possible to construct a zippered bag, instead of the coupling protrusion 11f' and the coupling hole 11g, to make the silicon 11d replaceable. Thus, if the silicon 11d has a replaceable structure, only the silicon 11d fixed to the support 11f can be replaced and used.

On the contrary, it is possible to form the barb-shaped coupling protrusion 11f' on the support 11f of the cup body 11b of the urine collecting cup 11 and to form the coupling hole 11g on the bottom surface of the silicon 11d.

The silicon 11d is configured such that its length is extended to climb over the opening 11a of the urine collecting cup 11, and has a structure of multiple-blade 11d' in which a plurality of silicon sheets overlap.

The silicon 11d may be configured such that a plurality of wrinkle bands 11s are formed on front surfaces of left and right sides of the cup body 11b and that a plurality of wrinkle strips 11t are formed around the opening of the silicon 11d.

As described above, since the silicon can be easily separated and replaced from the urine collecting cup, cost may be reduced as compared to purchasing a new urine collecting cup 11 as a whole. The coating area of viscous type of medical silicon which is coated on the front surface of the silicon 11d increases and thus it can be used by easily and conveniently adhering to the genital area. Also, it is possible to prevent dirt which is caught in the silicon coupling part, which allows hygienic management of the urine collecting cup 11.

Further, the cup body 11b is preferably configured as a transparent body to allow monitoring with the eye when adhering the urine collecting cup to the human perineum.

Meanwhile, the urine collection cup for men 11 comprises: a cup body 11' including an opening 11i into which the urine gathering unit 12 connected to the total conduit 13 is inserted on the upper part thereof and a thimble-shaped fastening part 11j on which a coupling protrusion ring is formed along an inner side surface on the bottom part; a wrinkled tube 11" in which one end thereof forms a coupling protrusion ring along an inner side surface to form a coupling ring hook 11k and the other end forms a tube into which a penis is inserted; and a coupling means 11a' for coupling the cup body 11' and the wrinkled tube 11".

The wrinkled tube 11" may form a through hole 11k' in a central portion of one side. It may also form a cutout portion 11k" in which an end portion of one side is cut out.

Also, it is preferable to prevent an offensive odor by installing a cover n' for covering an open portion of the wrinkled tube 11" provided on one side of a lower part of the wrinkled tube 11" through the hinge s'.

The coupling means 11a' comprises an upper groove 11b' formed for coupling to the thimble-shaped fastening part 11j along an upper external circumference, a lower groove 11b" formed for coupling to the ring hook 11k along a lower external circumference, a ring body 11c' between the upper and lower grooves 11b' and 11b", a girdle hanger 11d" formed along an external circumference of the ring body 11c' at regular intervals, and a total conduit fixture 11e' formed on one outer surface of the ring body 11c' to have a U-shape.

The tube 11k' is preferably configured such that a silicon thin film 11k" with a constant width is formed from the end portion to the inside, and a cutout portion 11m is formed in the thin film 11k".

As the cup body 11', a rubber or a soft material can be preferably used, but a hard material such as plastic can be used as well. The wrinkled tube 11" may form preferably a shape of a pleated cylinder by using latex or vinyl such as condom. Instead of the thin film 11k" of the cylindrical tube 11", a sticky type of medical silicon can be coated inside the end portion and adhered to the glans of the male penis. A girdle for men can be also used secondarily if necessary.

As the sensor attached to the sensor unit 12a, a capacitive sensor or an electrode-type sensor may be preferably used. It is preferable that the sensor line of the sensor unit 12a is connected via a flexible PCB inside the sensor unit 12a and the urine gathering cervix part 12b for a smooth rotation of the sensor unit 12a while the sensor line of the sensor unit 12a is connected between the urine gathering cervix parts 12b.

The total conduit 13 has a triple-pipe structure in which a Foley catheter 13a for draining the urine, a cleansing water pipe 13b for conveying the cleansing water, and a sensor pipe 13c for transmitting a signal of a sensor are separated inside one pipe.

Further, the total conduit 13 may have a double-pipe structure in which a sensor pipe 13c is independently separated and connected directly to the urine gathering unit 12 and the adaptor 14, and a Foley catheter 13a for draining the urine and a cleansing water pipe 13b for conveying a cleansing water are independently formed in the inside. Or, the total conduit 13 may be formed a single tube in which a sensor pipe 13c is separately divided and connected directly to the urine gathering unit 12 and the adaptor 14, and a Foley catheter 13a for draining the urine and a cleansing water pipe 13b for conveying a cleansing water are independently separated. The total conduit 13 has a structure capable of separating and replacing from the ends of the urine gathering unit 12 and the adaptor 14 to easily replace the total conduit 13, which is preferable for hygienic management. To achieve the object of hygienic replacement of each pipe, it is possible to separate all of the Foley catheter 13b, the cleansing water pipe 13b, and the sensor pipe 13c, which comprises the total conduit 13.

The adaptor 14 has a structure in which the urine gathering unit 12 is connected on the opposite end to which is connected to the total conduit 13 and is coupled to the coupling member 20a of the housing 20. The adaptor 14 comprises: an adaptor upper part 14a to which an end of the total conduit 13 is connected; a filtration unit 14b for filtering foreign materials in the urine; and an adaptor lower part 14c for fixing the filtration unit 14b.

In the adaptor upper part 14a, a Foley catheter opening 15b and a cleansing water pipe opening 15a are formed on the center of the bottom surface of the cylindrical space 15. A sensor jack 15c is installed on an exterior circumference of the adaptor upper part 14a. The Foley catheter opening 15b and the cleansing water pipe opening 15a are formed in a hollow portion in a bottom surface, and a locking means 15d is formed in a central portion.

Also, the filtration unit 14b comprises: a hollow portion 16 in which an outer peripheral portion of a circular plate body constituting a bottom surface forms a cylinder shape; a cleansing water pipe 13b' protruding inside and outside the hollow portion 16 in the bottom surface; a Foley catheter 13a' formed on the outside bottom surface of the hollow portion 16 to be protruded; and a filtering net 17, both left and right sides of which are adhered and fixed to the inside bottom surface of the hollow portion 16 with a regular spatial gap.

Preferably, a wing or a partition is installed such that the filtration unit 14b is inserted to the adaptor upper part 14a on a fixed position at all times.

The filtration unit 14b is preferably made with a transparent body to check a filtration state of the filtering net 17 for filtering foreign materials. It is preferable that a groove 15e is formed along an external circumference on the end portion of the cylindrical tub body that forms the space portion 15, a packing 15e' is inserted and installed in the groove 15e, grooves 15f and 15g are formed along external circumferences on the end portions of the Foley catheter 13a' and the cleansing water pipe 13b' that are formed and protruded on the outer bottom surface of the hollow portion 16, and packings 15f' and 15g' are inserted and installed in the grooves 15f and 15g to prevent leakage in the coupling portion of the adaptor upper part 14a and the filtering net 17 and the connecting portion to the coupling member 20a.

Since the urine storage unit 21 and the cleansing water storage unit 22 of the housing 20 are installed in spaces separated from each other and the opening of the urine storage unit 21 into which the urine storage tank 21a is inserted and the opening of the cleansing water storage unit 22 are not formed in the same direction, the possibility of contamination of the cleansing water stored in the cleansing water storage unit 22 with the urine in the urine storage tank 21a is fundamentally ruled out.

The urine storage tank 21a of the urine storage unit 21 comprises a body 21a' and a cap portion 21b and placed inside the housing. A groove 21t is formed in an upper surface of the body 21a', a sensor insertion groove 21s is formed on an inner end of the groove 21t, and a capacitive sensor 23d is installed to be protruded on a central portion of a bottom surface of the driving unit 23.

The best position of the deodorizing filter means 21c is a filter means reception space 21c' inside the housing as shown in Fig. 4a, even though it can be located in the urine storage tank as well.

The housing 20 may have two major embodiments according to the coupling manner of the urine storage unit. That is, there are a horizontal urine storage unit coupling type, as shown in Figs. 4a and 4b, and a vertical urine storage unit coupling type, as shown in Fig. 10.

The housing of the horizontal urine storage unit (21) suitable for floor heating type room which is placed and used on the floor. The vertical type may be conveniently used as a wall-mounted type when using the bed.

The horizontal type urine storage unit may have two major embodiments.

First, the urine storage tank 21a comprising the body 21a' and the cap portion 21b, as shown in Fig. 4b, is inserted into the housing in a horizontal direction and fastened by the coupling means 21e having a hinge 21e' and a locking ring 21e".

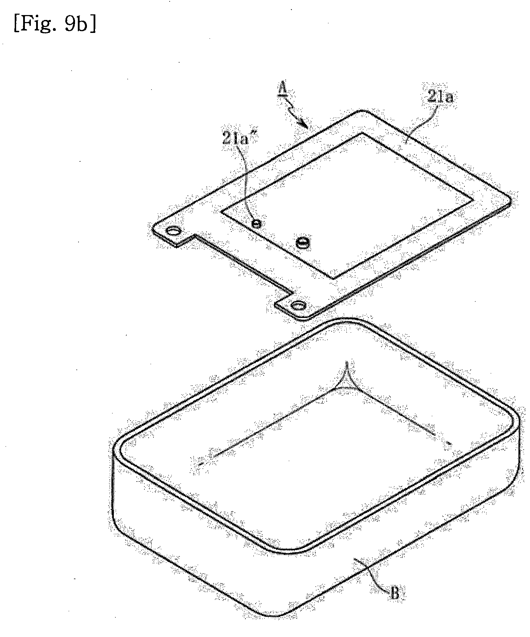

Second, the urine storage tank has a simple bowl B structure for receiving a disposable urine storage vinyl pack A and inserting into the housing in a horizontal direction (See Fig. 9b).

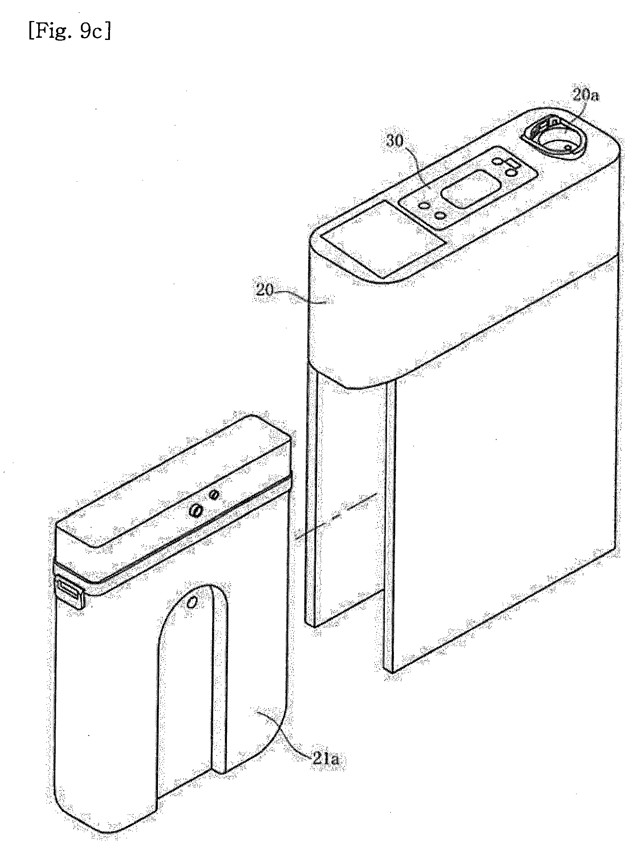

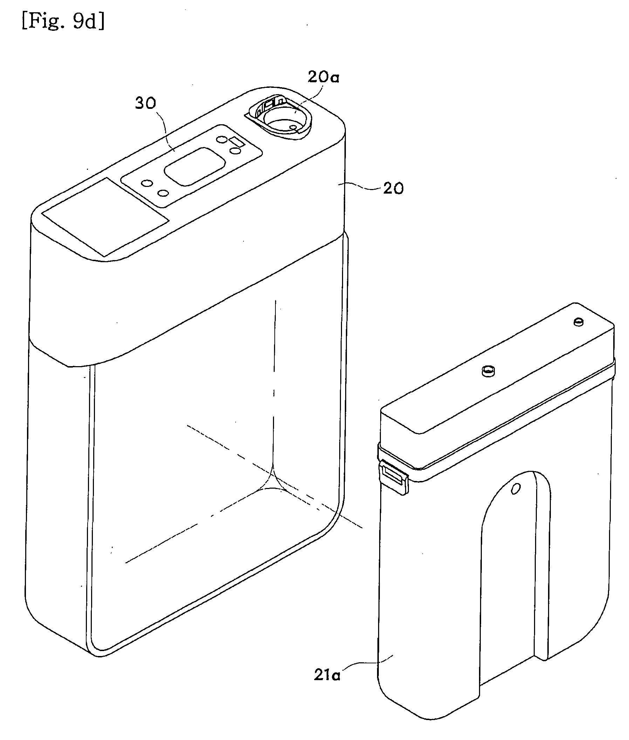

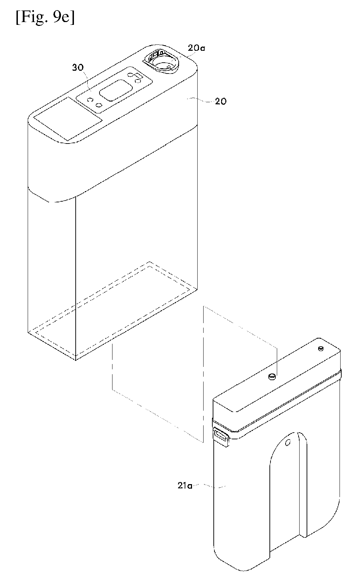

The vertical type urine storage unit housing has three major embodiments according to the coupling manner of the urine storage tank. That is, there are a forward and backward detachable type (Fig. 9c), a left and right (lateral) detachable type (Fig. 9d), and a upward and downward detachable type (Fig. 9e).

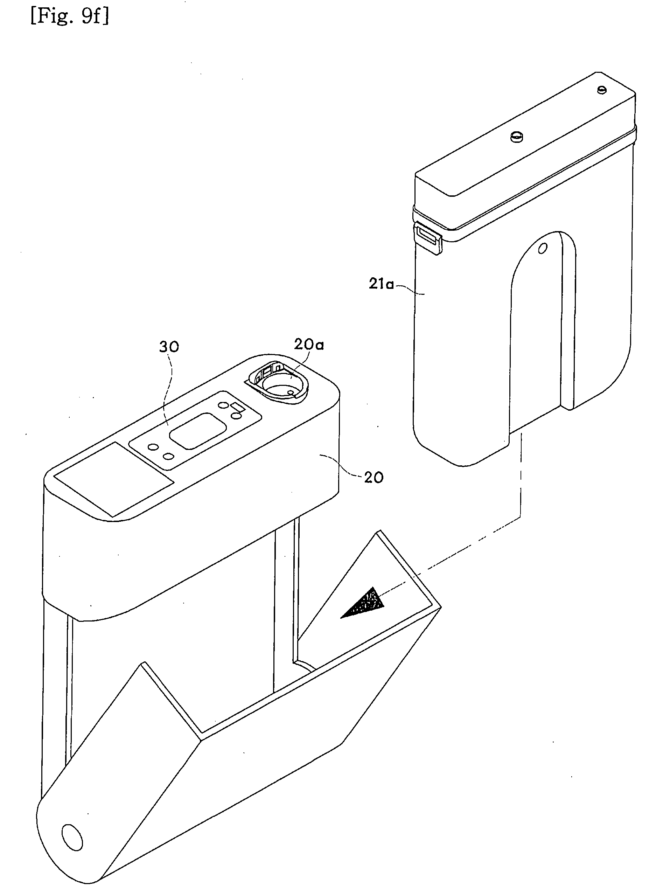

Among them, the most preferred embodiment is the left and right (lateral) detachable type, in which a cover of the urine storage unit is rotating relative to the bottom of the urine storage unit to open or shut the cover, the urine storage tank is inserted inside the opened cover, and the housing and the urine storage tank are automatically coupled when the cover is closed (Fig. 9f). Also, the urine storage tank 21a is installed in the housing 20 as a cover rotation type.

In the case of the vertical type urine storage unit housing, as shown in Fig. 9c, the urine storage tank may be made of a disposable vinyl pack to be detachable.

Preferably, the body 21a' of the urine storage tank 21a of the urine storage unit 21 and the disposable vinyl pack form a scale to measure the amount of the urine.

As described above, the deodorizing filter can be located in the cap portion of the urine storage tank of the urine storage unit, and the most preferred is to detachably locate within the housing.

Also, it is preferable that a temperature sensor 22a, a cleansing water filter 22', a water level sensor 22" and a hot plate 22b are installed on a bottom surface of the cleansing water storage unit 22 and that the temperature of the cleansing water may be maintained at a suitable temperature to clean the genital area by the temperature sensor 22a and the hot plate 22b. It is also preferable that an auxiliary cover is installed inside to prevent the cleansing water from undulating.

In this case, a ultrasonic generator means is provided on the bottom surface of the cleansing water storage unit 22 aside from the hot plate 22b to have a function of a humidifier. Further, the urine storage tank 21a of the urine storage unit 21 may have a bowl shape to accommodate a disposable vinyl pack. The body 21a' of the urine storage tank 21a of the urine storage unit 21 may form a scale to measure the amount of the urine. Preferably, a water level sensor 22" for detecting water level of the cleansing water is installed on the bottom surface of the cleansing water storage unit 22. The external flow of the cleansing water is prevented by the cleansing water storage unit cover 22f and the auxiliary cover.

A ceramic heat 22d and a bimetal 22e are installed on a lower part of the hot plate 22b, and it is preferable that a metal plate spring which has low thermal denaturation is installed on a lower part of the ceramic heat 22d to closely adhere the ceramic heat 22d to the hot plate 22b.

A temperature sensor 22c is installed on a bottom surface of the cleansing water storage unit 22 to heat the cleansing water and control a temperature suitable for the body temperature. When the temperature sensor 22c is not in a normal operation, a power supply to the ceramic heater 22d is shut down by the bimetal 22e which is installed to run parallel to control the temperature of the cleansing water to be below an appropriate temperature with no rick of low temperature burn to prevent a low temperature burn due to overheating of the ceramic heater 22d on the bottom portion of the hot plate 22b.

The cleansing water filter 22' comprises, as illustrated in Figs. 12a and 12b, a main body 22g having cylinder-shaped through holes 22a' and 22a"; a filtering net 22b' inserted and fixed to the main body 22g at a lower part of the through hole 22a"; an unidirectional shutter plate 22d' having a protrusion 22d" inserted into a groove 22c' formed in the main body 22g at a central upper part of the through hole 22a'; a shutter plate fixing ring 22e' inserted into the main body 22g above the shutter plate 22d' for fixing the protrusion 22d" of the shutter plate 22d' and for limiting an opening of the shutter plate 22d'; and a filter cover 22f' having a hollow portion 22f" for covering an upper surface of the main body 22g.

The driving unit 23 comprises: a urine intake pump 23a for moving the urine in the urine gathering unit 12 to the urine storage tank 21a of the urine storage unit 21 through a urine hose; a cleansing water pump 23b for moving the cleansing water stored in the cleansing water storage unit 22 to the cleansing water pipe 13b of the total conduit 13 through a cleansing water hose; and a drying pump 23c for supplying dry air to the cleansing water pipe 13b of the total conduit 13 through the cleansing water hose.

Since the cleansing water hose is used as passages for both the cleansing water and the dry air, it is preferable that a T pipe is used at its connection part.

The control unit 24 comprises a microprocessor to control an operation of each part of the automatic urine collecting apparatus of the present invention according to a preset program by a manipulation setting of the operation panel 30, and the manipulation state and the operational state of each part are displayed on a display screen of the operation panel 30.

Further, the control unit 24 may inform the manipulation state and the operational state of each part by voice through a speaker or may send out music or other sound.

The filtration unit 25 comprises: a tub body 25a in which a deodorizing filter incorporated; a urine backflow blocking means 25b installed to a through hole 21a" connected to the urine storage tank 21a; and a plurality of air outlets 25c and it is to remove an offensive odor of the air pushed out from the urine storage tank 21a in the course that the urine fills the urine storage tank 21a of the urine storage unit 21. It comprises a plurality of partitioning plates 25a' placed in zigzag to increase the surface area of the filter to maximize the filter function; a filter filler inserted in the space made by the tub body 25a and the partitioning plates 25a'; a urine backflow blocking means 25b installed detachable to a through hole 21a" through which the air having the offensive odor is pushed out from the urine storage tank 21a; and a plurality of air outlets 25c.

The urine backflow blocking means 25b comprises a cylindrical tub body 25b' having a hollow portion therein and a moving ball 25b" inserted into the cylindrical tub body 25b'. In the case that the urine collecting apparatus is shaking or it is installed with inclination, the urine may undulate to cause a backflow of the urine to the urine through hole 21a' of the storage tank 21a. Then, the moving ball 25b" floats by the urine flowed back to block an opening 25f of an the cylindrical tub body of an upper part of the urine backflow blocking means 25b.

The moving ball 25b" consists of an upper sphere body 25d and wings 25e formed on a lower side to have a regular angular interval radially.

As the deodorizing filter, any one or at least one of bio filter, activated carbon fiber filter, and charcoal filter may be used. It is preferable that the structure of the filter is modulated to be easily attached or detached in the housing 20 or the urine storage tank 21a, which makes a replacement of the filter convenient.

Further, the housing sensor unit designates a capacitive sensor 21d built within the housing 20 for detecting the center portion of the upper surface of the body 21a' to detect the fullness of the urine; a cleansing water level sensor installed on the bottom surface of the cleansing water storage unit 22 for detecting water level of the cleansing water to detect whether the cleansing water is in shortage; and a temperature sensor is installed within the cleansing water storage tank 22a as the housing sensor unit installed on the bottom of the cleansing water storage unit 22 to detect a temperature of the cleansing water.

The water level sensor that may be used includes any one of a capacitive sensor, electrode-type sensor, optical sensor, and magnetic field sensor.

The operation panel 30 is a touchscreen, which comprises: a display screen 31 for displaying a manipulation state and an operational state of each part; an automatic/manual cleaning button 32 and 32'; a hot water/drying button 33 and 33'; a continuous cleaning selection button 34; a measurement reset button 35; a measurement mode change button 36; and a nighttime mode button.

The display screen 31 displays the number of urination and the amount of the cleansing water as well as abnormalities of the automatic urine collecting apparatus of the present invention and warning messages. Preferably, it may be configured to emit a warning sound or informational voice message and to play natural sound and various music during intake of the urine as well.

The present invention can further provides a wheelchair mounting means 40 to mount the automatic urine collecting apparatus of the present invention on a wheelchair, wherein the wheelchair mounting means 40 comprises a tub body 41 for accommodating the automatic urine collecting apparatus of the present invention, which includes a bottom plate 41a and a wall body 41b; belt holes 42 formed in the periphery of the tub body 41 at regular intervals; and a plurality of belts inserted into the belt holes 42 and tied to the wheelchair; and further comprises a tool reception unit 43 for receiving articles which is separated from the tub body 41.

Also, the present invention provides a rechargeable battery within the housing 20 for allowing the use of the apparatus where power supply is not provided.

An X girdle for women G which is used for wearing the urine collecting cup 11 of the urine collecting unit 10 according to the present invention comprises, as shown in Fig. 18a, a waist band C including a plurality of band rings a formed on a front surface of left and right sides, Velcro tapes b and b' formed on the front surface of one end and a rear surface of the other end, respectively, and X band fastening button b" formed on the front surface of the one end and having pads p and p' under the rear surface; an X band K including back straps d and d' having fastening means c and c' on their ends, front straps f and f' having fastening means e and e' on their ends, a support strip g connected between the back straps d and d', a hose securing button installation strip h connected between the front straps f and f', a button insertion band i which is formed in the hose securing button installation strip h and has a plurality of fastening button insertion holes in longitudinal direction, and a hose securing button j fixed to one end of the button insertion band i, thereby the back straps d and d' and the front straps f and f' are cross-connected in an X-shape.

On the other hand, an X girdle for men G is substantially the same as the above-described X girdle for women G, as shown in Fig. 19a, except for the configuration of the intersection where the back straps d and d' and the front straps f and f' are cross-connected in an X-shape. Accordingly, detailed description of the parts having the same configuration is omitted, and only the different parts will be described below.

That is, in the X girdle for men G, the intersection m where the back straps d and d' and the front straps f and f' are crossing each other to make an X-shape is formed such that an upper part of which is narrower and a lower part of which is broader, a hole n is formed on a center of the intersection m, a double-layered fabric is formed on the intersection m at an upper outer part of the hole n, rings o hooked to a girdle hanger 11d" of a urine collecting cup for men 11 are formed along an external circumference at an inner surface of the hole n at regular intervals, and a button insertion band i having a plurality of fastening button insertion holes and a hose securing button j fixed to one end of the button insertion band i are formed on the intersection m below the hole n.

Next, detailed description of the operation and effect of the automatic urine collection apparatus of the present invention which is configured as described above will be given.

The automatic urine collection apparatus of the present invention is mostly used for patients with physical disabilities. First, the urine collecting cup 11 of the urine collecting unit 10 is placed on the pubic regions of a user (female), as shown in Fig. 17, the girdle X is worn to secure the urine collecting cup 11 to the genital regions of the user (female), and a stool bag Y is attached to the skin around the anal regions.

According to the presence of the sticky type medical silicon coating on the surface of the silicon of the urine collecting cup contacting the genital regions of the user, it is determined whether or not to wear the girdle X. That is, if the sticky type of medical silicon is not coated, it is preferably used by the patient who can be aware of the urination so that the patient grabs the urine collecting unit and put it to the genital regions by himself/herself when urinating. In this case, the patient does not need to wear the girdle X.

However, if the user is not aware of the urination such as the case of the incontinence of urine, the urine collecting unit should be contacted to the genital regions of the user constantly. Accordingly, it is preferable that the sticky type of medical silicon is coated on the silicon, and the girdle can be used secondarily if necessary.

Also, as shown in Figs. 6a-6b, a plurality of silicon sheets 11d may be used to make a multiple-blade 11d' structure. In the case of the multiple blades, the sticky type medical silicon may be coated using each blade as a border.

Further, as shown in Figs. 18a and 18b, when using the X girdle for women G, let each back strap d and d' pass through both the inner band ring a and the outer band ring a on both sides, turn over and spread the waist band C, lay the patient down such that the central portion of the buttocks of the patient is located on the center of the intersection of the X band K, stick the Velcro tape b and b' to tighten the waist band C, fasten back straps d and d' using the fastening means c and c', pull and adjust the ends of the back straps d and d', and pull the front straps f and f' up to the abdomen side. Then, let each of the front straps f and f' pass through the band ring a which moved inwardly from both ends by the fastening of the waist band C, fasten them using the fastening means e and e', and pull and adjust the ends of the front straps f and f'. At this time, the front straps f and f' are adjusted for the intersection of the X band K to exactly cover the pubic regions. In this state, insert the urine collecting cup 11 between the intersection of the X band K and the hose securing button installation strip h to locate the aperture of the silicon 11d of the urine collecting cup 11 precisely on the pubic regions, adjust the back straps d and d' and the front straps f and f' more, insert and couple the urine gathering unit 12 to the opening 11a of the urine collecting cup 11, and secure the hose by the hose securing button j. Then, it is ready for use.

Meanwhile, in the case of a male, as shown in Figs. 19a and 19b, it is the same as the wearing state of the X girdle for women G as described above except that the ring o of the X girdle G is hanged on the girdle hanger 11d" of the urine collecting cup for men 11 and the penis is inserted into the tube 11k' of the urine collecting cup 11. Accordingly, detailed description thereof is omitted.

Now, the adaptor 14 is connected to the housing coupling member 20a, and then the operation of the automatic urine collecting apparatus of the present invention is started.

When the user urinates, the urine is gathered in the urine gathering unit 12 of the urine collecting unit 10, a urine detecting sensor detects the gathered urine and sends a detection signal to the control unit 24, the control unit 24 determines that the user urinated by the detection signal and operates the driving unit 23 according to a preset program in a microprocessor in the control unit 24, and the urine is sucked through the Foley catheter 13a of the total conduit 13 and moved to the urine storage unit 21.

When there is no urine left in the urine gathering unit 12, the urine detecting sensor detects it and stops the operation of the urine intake driving unit through the control unit 24. Then, the control unit 24 starts the cleansing water driving unit to move the cleansing water through the cleansing water pipe 13b of the total conduit 13 and spray it through the opening 12d of the urine gathering cervix part 12b to wash the pubic regions. The urine detecting sensor again detects the sprayed cleansing water and it is sucked into the Foley catheter 13a of the total conduit 13 and moved to the urine storage tank 21a of the urine storage unit 21 under the control of the control unit 24. After driving of the cleansing water is finished, a drying driving unit is activated and dries the pubic regions. Through the above processes, the urine collecting apparatus operates automatically.

The cleansing water filtered by the deodorizing filter 22' as shown in Figs. 12a and 12b is used as the cleansing water, and the cleansing water flows in a direction of the arrow shown in Fig. 12b and filtered. That is, the cleansing water filtered through the filtering net 22b' flows through the hollow portion 22f" and an aperture of a shutter plate fixing ring 22e' and passes through the opened shutter plate 22d'. At this time, the shutter plate 22d' is opened as indicated in two dot chain line when the cleansing water flows in the direction of the arrow; however, if it flows in the opposite direction, i.e., it flows upward, the shutter plate 22d' stays closed being caught by the shutter plate fixing ring 22e', which prevents the backflow of the cleansing water.

Further, the cleansing water hose is used as a hose supplying dry air as well.

In the automatic urine collection apparatus of the present invention which is operating as described above, the manipulation state and the operational state of each part are displayed on the display screen 31 of the operational panel 30, which allows the user to understand the state thereof upon watching the display screen 31. That is, the user touches an appropriate button on the operational panel 30 to start the automatic urine collection apparatus. For example, if the user touches the automatic cleaning button 32, a series of the processes described above are performed automatically. If the automatic cleaning is not activated, only the urine intake feature during urination is performed. Also, if the user touches the manual cleaning button 32', the cleaning feature is executed at any time. If the hot water feature is activated, the cleansing water is heated to the temperature appropriate for the body temperature by the ceramic heater. Through the drying button 33', strength of the drying feature is adjusted according to the season, and the operational panel 30 having these various buttons is preferably implemented as a touchscreen.

The urine level detecting sensor installed inside the housing 20 for detecting the center portion of the upper surface of the body 21a' of the urine storage unit 21 is preferably a capacitive sensor 21d. The urine level detecting sensor 21d detects the urine level, and recognizes if the urine storage tank 21a is full of urine. When it is full of urine, the display screen 31 of the operational panel 30, or a speaker, or both of them warn to empty the urine storage tank 21a. Due to this feature, the user may empty the urine storage tank 21a properly before it overflows and use it again.

Further, the cleansing water level sensor installed on the bottom surface of the cleansing water storage unit 22 detects the water level of the cleansing water and indicates the detection result through the display screen 31 or a speaker, or both of them to warn the user. Accordingly, the user learns the shortage of the cleansing water in the cleansing water storage unit 22 and replenishes the cleansing water properly.

In addition, the temperature sensor installed on the bottom of the cleansing water storage unit 22 measures the temperature regularly and automatically control the heating rate of the ceramic heater 22d through the control unit 24. If the temperature sensor is not in a normal operation and cannot control the heating of the ceramic heater 22d automatically, power supply to the ceramic heater 22d is shut down by the bimetal 22e installed to be run parallel to prevent the risk of low-temperature burn due to overheating of the cleansing water.

The present invention has industrial applicability in that it allows a bedridden patient who cannot use the bathroom for urination to conveniently use, maintains a sanitary condition, relieves mental and economic burdens, and cleans the urine storage tank and the cleansing water storage tank easily.

The present invention is described in detail with reference to preferred embodiments, but the present invention is not limited to the above specific embodiments. Rather, various changes and modifications can be made to the invention without departing from the scope of the present invention.

### [Description of Reference Numerals]

10 : urine collecting unit 11 : urine collecting cup
11': cup body 11" : cylindrical tube
11a : opening (for women) 11a' : coupling means
11b : cup body 11c: rim
11c' : groove 11d : silicon
11d' : multiple blade 11e : fixing means
11f : support 11f' : coupling protrusion
11g : silicon support 11g' : coupling hole
11i : opening (for men) 11j : fastening part
11k : ring hook 1k' : tube
1b' : upper groove 11b" : lower groove
11c' : ring body 11d" : girdle hanger
11e' : total conduit fixture 11s : wrinkle bands
11t : wrinkle strips 12 : urine gathering unit
12a : urine sensor unit 12b : urine gathering cervix part
12c : urine gathering corpus part 12d : cleansing water spray opening
13 : total conduit 13a : Foley catheter
13b : cleansing water pipe 13c : sensor pipe
14 : adaptor 14a : adaptor upper part
14b : filtration unit 14c : adaptor lower part
15 : cylindrical space 15a : Foley catheter opening
15b : cleansing water pipe opening 15c : sensor jack
15d : locking means 15e : groove
15e' : packing 15f, 15g : groove
15f', 15g' : packing 16 : hollow portion
20 : housing 20a : coupling member
21 : urine storage unit 21a : urine storage tank
21a' : body 21a" : through hole
21b : cap portion 21c : filter means
21c' : filter means reception space 21d : capacitive sensor
21e : coupling means 21e' : hinge
21e" : locking ring 21t : double groove
21s : sensor insertion groove 22 : cleansing water storage unit
22' : cleansing water filter 22" : water level sensor
22a : temperature sensor 22b : hot plate
22c : auxiliary cover 22d : ceramic heater
22e : bimetal 22e' : shutter plate fixing ring
22f : cleansing water storage unit cover 22f' : filter cover
22f" : hollow portion 22g : main body
22a', 22a" : through hole 22b' : filtering net
22c' : groove 22d' : shutter plate
22d" : protrusion 23 : driving unit
23a : urine intake pump 23b : cleansing water pump
23c : drying pump 23d : capacitive sensor
24 : control unit 25 : filtration unit
25a' : partitioning plate 25a : tub body
25b : urine backflow blocking means 25b' : cylindrical tub body
25b" : moving ball 25c : air outlet
25d : spherical body 25e : wing
30 : operation panel 31 : display screen 32 : automatic cleaning button 32' : manual cleaning button 33 : hot water button 33' : drying button
34 : continuous cleaning selection button 35 : measurement reset button
36 : measurement mode button 40 : wheelchair mounting means
41 : tub body 41a : bottom plate
41b : wall body 42 : belt hole
43 : tool reception unit A : urine storing vinyl pack
B : bowl C : waist band
G : X girdle K : X band
X : girdle Y : stool bag
a : band ring b, b' : Velcro tape
c, c' : fastening means d, d' : back strap
e, e' : fastening means f, f' : front strap
g : support strip h : hose securing button installation strip
i : button insertion band j : hose securing button
m : intersection n : hole
o : ring p, p' : pad
s, s' : hinge t, t' : cover

## Claims

1. An automatic urine collection apparatus which comprises:
a urine collecting unit 10 comprising a urine collecting cup 11 for collecting urine from a user, a urine gathering unit 12 for recognizing and gathering the urine and spraying a cleansing water to a urination part, an adaptor 14 for connecting to a device, and a total conduit 13;
a housing 20 comprising a urine storage unit 21 for storing the urine collected from the urine collecting unit 10, a cleansing water storage unit 22 for storing the cleansing water used in the urine collecting unit 10, a driving unit 23 for moving the urine in the urine collecting unit 10 to the urine storage unit 21 and moving the cleansing water in the cleansing water storage unit 22 to the urine collecting unit 10, a control unit 24 for controlling an operation of the driving unit 23, a filtration unit 25, and a housing sensor unit including sensors for detecting a urine level of the urine storage unit 21, a cleansing water level of the cleansing water storage unit 22, and a temperature of the cleansing water; and
an operational panel 30 for manipulating a control of the control unit 24 and checking its state.

2. The automatic urine collection apparatus of claim 1, wherein the urine gathering unit 12 comprises: a urine gathering cervix part 12b comprising a structure for attaching or detaching the urine collecting cup 11 by being inserted into an opening 11a of the urine collecting cup 11 for gathering the spouted urine and an opening 12d for supporting a sensor unit 12a to be rotatable and for spraying the cleansing water; and a urine gathering corpus part 12c connected to the total conduit 13.

3. The automatic urine collection apparatus of claim 1, wherein the urine collecting cup 11 is configured such that a cup body 11b, which has an opening 11a for gathering urine on an upper part, a lower part of which has a semicircular shape, and a front surface of which is opened, to which a fixing means 11e of a ring shape is inserted and adhered; and a silicon 11d, a center portion of which is opened, is inserted and fused, to secure a rotatable space for the sensor unit (12a), thereby the cup body 11b, the silicon 11d and the fixing means 11e are integrally used for women.

4. The automatic urine collection apparatus of claim 3, wherein the cup body (11b) is transparent.

5. The automatic urine collection apparatus of claim 3, wherein an offensive odor is prevented by installing a cover (t) for covering the front open portion of the cup body 11b through a hinge (s) provided on an upper part of the fixing means 11e.

6. The automatic urine collection apparatus of claim 3, wherein a length of the silicon 11d is configured such that its length is extended to climb over the opening 11a of the urine collecting cup 11, and has a structure of multiple-blade 11d' in which a plurality of silicon sheets overlap.

7. The automatic urine collection apparatus of claim 3, wherein the silicon 11d is configured such that a plurality of wrinkle bands 11s are formed on front surfaces of left and right sides of the cup body 11b, and a plurality of wrinkle strips 11t are formed around the opening of the silicon 11d.

8. The automatic urine collection apparatus of claim 3, wherein a coupling hole 11g is in a support 11f of the cup body 11b of the urine collecting cup 11, and a coupling protrusion 11f' is on a bottom surface of the silicon (11d), which makes the silicon 11d replaceable in forward and backward directions.

9. The automatic urine collecting apparatus of claim 3, wherein a coupling hole 11g is in a support 11f of the cup body 11b of the urine collecting cup 11, and a coupling protrusion 11f' is on a bottom surface of the silicon 11d, which makes the silicon 11d replaceable in a lateral direction.

10. The automatic urine collection apparatus of claim 8 or claim 9, wherein the coupling hole 11g and the coupling protrusion 11f' are formed to have barb shapes, which are not to be easily missed after the coupling protrusion 11f' is inserted laterally.

11. The automatic urine collection apparatus of claim 8 or claim 9, wherein the coupling protrusion is formed to have a barb shape on the support 11f of the cup body 11b of the urine collecting cup 11, and the coupling hole is formed on the lower surface of the silicon 11d, which are not to be easily missed after the coupling protrusion 11f' is inserted laterally.

12. The automatic urine collection apparatus of claim 1 or claim 2, wherein the urine collecting cup 11 comprises: a cup body 11' including an opening 11i into which the urine gathering unit 12 connected to the total conduit 13 is inserted on the upper part thereof and a thimble-shaped fastening part 11j on which a coupling protrusion ring is formed along an inner side surface on the bottom part; a wrinkled tube 11" in which one end thereof forms a coupling protrusion ring along an inner side surface to form a coupling ring hook 11k and the other end forms a tube into which a penis is inserted; and a coupling means 11a' for coupling the cup body 11' and the wrinkled tube 11, and is used for men.

13. The automatic urine collection apparatus of claim 12, wherein the coupling means 11a' comprises: an upper groove 11b' formed for coupling to the thimble-shaped fastening part 11j along an upper external circumference, a lower groove 11b" formed for coupling to the ring hook 11k along a lower external circumference, a ring body 11c' between the upper and lower grooves 11b' and 11b", a girdle hanger 11d" formed along an external circumference of the ring body 11c' at regular intervals, and a total conduit fixture 11e' formed on one outer surface of the ring body 11c' to have a U-shape.

14. The automatic urine collection apparatus of claim 12, wherein the wrinkled tube 11" has a through hole 11k' formed in a central portion of one side.

15. The automatic urine collection apparatus of claim 12, wherein the wrinkled tube 11" has a cutout portion (11k") in which an end portion of one side is cut out.

16. The automatic urine collection apparatus of claim 12, wherein a hinge (s') is installed on one side of a lower part of the wrinkled tube 11" and a cover n' for covering an open portion of the wrinkled tube 11" is installed through the hinge (s') to prevent an offensive odor.

17. The automatic urine collection apparatus of claim 12, wherein the cup body 11' is made with a rubber or a soft material.

18. The automatic urine collection apparatus of claim 12, wherein the cup body 11' is hard plastic.

19. The automatic urine collection apparatus of claim 12, wherein the wrinkled tube 11" is made with latex or vinyl such as silicon or condom harmless to humans.

20. The automatic urine collection apparatus of claim 3, wherein the sensor attached to the sensor unit 12a is a capacitive sensor.

21. The automatic urine collection apparatus of claim 3, wherein the sensor attached to the sensor unit 12a is an electrode-type sensor.

22. The automatic urine collection apparatus of claim 3, wherein a sensor line of the sensor unit 12a is connected via a flexible PCB inside the sensor unit 12a and the urine gathering cervix part 12b for a smooth rotatation of the sensor unit 12a while the sensor line of the sensor unit 12a is connected between the urine gathering cervix part 12b.

23. The automatic urine collection apparatus of claim 1, wherein the total conduit 13 has a triple-pipe structure in which a Foley catheter 13a for draining the urine, a cleansing water pipe 13b for conveying the cleansing water, and a sensor pipe 13c for transmitting a signal of a sensor are separated inside one pipe, but three pipes are separated, respectively.

24. The automatic urine collection apparatus of claim 1, wherein the total conduit 13 has a double-pipe structure in which a sensor pipe 13c is independently separated and connected directly to the urine gathering unit 12 and the adaptor 14, and a Foley catheter 13a for draining the urine and a cleansing water pipe 13b for conveying a cleansing water are independently formed in the inside.

25. The automatic urine collecting apparatus of claim 1, wherein the total conduit 13 is formed of a single tube in which a sensor pipe 13c is separately divided and connected directly to the urine gathering unit 12 and the adaptor 14, and a Foley catheter 13a for draining the urine and a cleansing water pipe 13b for conveying a cleansing water are independently separated.

26. The automatic urine collection apparatus of claim 24, wherein the total conduit 13 has a structure capable of separating and replacing from the ends of the urine gathering unit 12 and the adaptor 14.

27. The automatic urine collection apparatus of claim 25, wherein the total conduit 13has a structure capable of separating and replacing from the ends of the urine gathering unit 12 and the adaptor 14.

28. The automatic urine collection apparatus of claim 1, wherein the adaptor 14 comprises: an adaptor upper part 14a to which an end of the total conduit 13 is connected; a filtration unit 14b for filtering foreign materials in the urine; and an adaptor lower part 14c for fixing the filtration unit 14b.

29. The automatic urine collection apparatus of claim 28, wherein a sensor jack 15c is installed on an exterior circumference of the adaptor upper part 14a, a Foley catheter opening 15b and a cleansing water pipe opening 15a are formed in a hollow portion in a bottom surface, and a locking means 15d is formed in a central portion.

30. The automatic urine collection apparatus of claim 28, wherein the filtration unit 14b comprises: a hollow portion 16 in which an outer peripheral portion of a circular plate body constituting a bottom surface forms a cylinder shape; a cleansing water pipe 13b' protruding inside and outside the hollow portion 16 in the bottom surface; a Foley catheter 13a' formed on the outside bottom surface of the hollow portion 16 to be protruded; and a filtering net 17, both left and right sides of which are adhered and fixed to the inside bottom surface of the hollow portion 16 with a regular spatial gap.

31. The automatic urine collection apparatus of claim 28, wherein a wing or a partition is installed such that the filtration unit 14b is inserted to the adaptor upper part 14a on a fixed position at all times.

32. The automatic urine collection apparatus of claim 28, wherein the filtration unit 14b is made with a transparent body to check a filtration state of the filtering net 17 for filtering foreign materials.

33. The automatic urine collection apparatus of claim 1, wherein the urine storage unit 21 and the cleansing water storage unit 22 of the housing 20 are installed in spaces separated from each other.

34. The automatic urine collection apparatus of claim 1, wherein the urine storage tank 21a of the urine storage unit 21 comprises a body 21a' and a cap portion 21b.

35. The automatic urine collection apparatus of claim 34, wherein a groove 21t is formed in an upper surface of the body 21a', a sensor insertion groove 21s is formed on an inner end of the groove 21t, and a capacitive sensor 23d is installed to be protruded on a central portion of a bottom surface of the driving unit 23.

36. The automatic urine collection apparatus of claim 1 or claim 33, wherein the urine storage tank (21a) of the urine storage unit 21 has a bowl shape to accommodate a disposable vinyl pack.

37. The automatic urine collection apparatus of claim 1 or claim 33, wherein a coupling means for coupling to the body 21a' is installed on a cap portion 21b of the urine storage tank 21a of the urine storage unit 21.

38. The automatic urine collection apparatus of claim 1 or claim 33, wherein a body 21a' of the urine storage tank 21a of the urine storage unit 21 forms a scale to measure an amount of the urine.

39. The automatic urine collection apparatus of claim 1, wherein a temperature sensor 22a, a cleansing water filter 22', a water level sensor 22" and a hot plate 22b are installed on a bottom surface of the cleansing water storage unit 22.

40. The automatic urine collection apparatus of claim 39, wherein an ultrasonic generator means is provided on the bottom surface of the cleansing water storage unit 22 aside from the hot plate 22b to have a function of a humidifier.

41. The automatic urine collection apparatus of claim 1, wherein the external flow of the cleansing water is prevented by the cleansing water storage unit cover 22f of the housing 20.

42. The automatic urine collection apparatus of claim 39, wherein a ceramic heat 22d and a bimetal temperature sensor 22e are installed on a lower part of the heat plate 22b.

43. The automatic urine collection apparatus of claim 39, wherein a temperature sensor 22c is installed on a bottom surface of the cleansing water storage unit 22 to control a temperature of the cleansing water by the hot plate 22b, and, when the temperature sensor 22c is not in a normal operation, a power supply to the ceramic heat is shut down by the bimetal 22e to prevent a low temperature burn due to overheating of the heat plate 22b.

44. The automatic urine collection apparatus of claim 39, wherein the cleansing water filter 22' comprises: a main body 22g having cylinder-shaped through holes 22a' and 22a"; a filtering net 22b' inserted and fixed to the main body 22g at a lower part of the through hole 22a"; an unidirectional shutter plate 22d' having a protrusion 22d" inserted into a groove 22c' formed in the main body 22g at a central upper part of the through hole 22a'; a shutter plate fixing ring 22e' inserted into the main body 22g above the shutter plate 22d' for fixing the protrusion 22d" of the shutter plate 22d' and for limiting an opening of the shutter plate 22d'; and a filter cover 22f' having a hollow portion 22f" for covering an upper surface of the main body 22g.

45. The automatic urine collection apparatus of claim 1, wherein the driving unit 23 comprises: a urine intake pump 23a for moving the urine in the urine gathering unit 12 to the urine storage tank 21a of the urine storage unit 21 through a urine hose; a cleansing water pump 23b for moving the cleansing water stored in the cleansing water storage unit 22 to the cleansing water pipe 13b of the total conduit 13 through a cleansing water hose; and a drying pump 23c for supplying dry air to the cleansing water pipe 13b of the total conduit 13 through the cleansing water hose.

46. The automatic urine collection apparatus of claim 41, wherein the cleansing water hose has a T pipe at its connection part since it is used as passages for both the cleansing water and the dry air.

47. The automatic urine collection apparatus of claim 1, wherein the control unit 24 comprises a microprocessor to control an operation of each part of the automatic urine collection apparatus according to a preset program by a manipulation setting of the operational panel 30.

48. The automatic urine collection apparatus of claim 1, wherein a manipulation state and an operational state of each part are displayed on a display screen of the operation panel 30 under a control of the control unit 24.

49. The automatic urine collection apparatus of claim 1, wherein the control unit 24 informs a manipulation state and an operational state of each part by voice through a speaker or sends out music or other sound.

50. The automatic urine collection apparatus of claim 1, wherein the filtration unit 25 comprises: a tub body 25a having a deodorizing filter therein; a urine backflow blocking means 25b installed to a through hole 21a" connected to the urine storage tank 21a; and a plurality of air outlets 25c.

51. The automatic urine collection apparatus of claim 50, wherein the urine backflow blocking means 25b comprises a cylindrical tub body 25b' having a hollow portion therein and a moving ball 25b" inserted into the cylindrical tub body 25b' such that the moving ball 25b" floats to block an opening 25f of an upper part of the cylindrical tub body by the urine flowed back when the urine in the urine storage tank 21a flows back to the through hole 21a".

52. The automatic urine collection apparatus of claim 51, wherein the moving ball 25b" comprises an upper spherical body 25d and wings 25e formed on a lower side to have a regular angular interval radially.

53. The automatic urine collection apparatus of claim 50, wherein the filtration unit includes at least one of bio filter, activated carbon fiber filter, and charcoal filter as fillers.

54. The automatic urine collection apparatus of claim 1, wherein a structure of the filtration unit 25 is modulated to be easily attached or detached in the housing 20 which makes a replacement of the filter convenient.

55. The automatic urine collection apparatus of claim 1, wherein a capacitive sensor 21d is built within the housing 20 as the housing sensor unit to detect the fullness of the urine.

56. The automatic urine collection apparatus of claim 1, wherein a water level sensor for detecting water level of the cleansing water is installed in the cleansing water storage unit 22 as the housing sensor unit to detect whether the cleansing water is in shortage.

57. The automatic urine collection apparatus of claim 56, wherein the water level sensor is any one of a capacitive sensor, electrode-type sensor, optical sensor, and magnetic field sensor.

58. The automatic urine collection apparatus of claim 1, wherein a temperature sensor is installed within the cleansing water storage tank 22a as the housing sensor unit to detect a temperature of the cleansing water.

59. The automatic urine collection apparatus of claim 1, wherein the operational panel 30 comprises: a display screen 31 for displaying a manipulation state and an operational state of each part; an automatic/manual cleaning button 32, 32'; a hot water/drying button 33, 33'; a continuous cleaning selection button 34; a measurement reset button 35; a measurement mode change button 36; and a nighttime mode button.

60. The automatic urine collection apparatus of claim 1, wherein the operational panel 30 comprises a touchscreen.

61. The automatic urine collection apparatus of claim 59, wherein the display screen 31 displays the number of urination and the amount of the cleansing water as well as abnormalities of the automatic urine collection apparatus and warnings.

62. The automatic urine collection apparatus of claim 1, further comprising a wheelchair mounting means 40 which includes: a tub body 41 for accommodating the automatic urine collection apparatus including a bottom plate 41a and a wall body 41b; belt holes 42 formed in a periphery of the tub body 41 at regular intervals; and a plurality of belts inserted into the belt holes 42 and tied to a wheelchair; and which further comprises a tool reception unit 43 for receiving articles, which is separated from the tub body 41.

63. The automatic urine collection apparatus of claim 1, wherein an X girdle for women (G) which is used for wearing the urine collecting cup 11 of the urine collecting unit 10 comprises:
a waist band (C) including a plurality of band rings (a) formed on a front surface of left and right sides, Velcro tapes (b, b') formed on the front surface of one end and a rear surface of the other end, respectively, and X band fastening button (b") formed on the front surface of the one end and having pads (p, p') under the rear surface; and
an X band (K) including back straps (d, d') having fastening means (c, c') on their ends, front straps (f, f') having fastening means (e, e') on their ends, a support strip (g) connected between the back straps (d, d'), a hose securing button installation strip (h) connected between the front straps (f, f'), a button insertion band (i) which is formed in the hose securing button installation strip (h) and has a plurality of fastening button insertion holes in longitudinal direction, and a hose securing button (j) fixed to one end of the button insertion band (i) such that the back straps (d, d') and the front straps (f, f') are cross-connected in an X-shape.

64. The automatic urine collection apparatus of claim 63, wherein the X band (K) for women is made with pleated felt other than fabrics which is conveniently usable as disposable.

65. The automatic urine collection apparatus of claim 63, wherein an intersection (m) where the back straps (d, d') and the front straps (f, f') are crossing each other to make an X-shape is formed such that an upper part of which is narrower and a lower part of which is broader, a hole (n) is formed on a center of the intersection (m), a double-layered fabric is formed on the intersection (m) at an upper outer part of the hole (n), rings (o) hooked to a girdle hanger (11d") of a urine collecting cup for men (11) are formed along an external circumference at an inner surface of the hole (n) at regular intervals, and a button insertion band (i) having a plurality of fastening button insertion holes and a hose securing button (j) fixed to one end of the button insertion band (i) are formed on the intersection (m) below the hole (n) to use it as an X girdle for men (G).

66. The automatic urine collection apparatus of claim 63, wherein the X band (K) for men is made with pleated felt other than fabrics which is conveniently usable as disposable.

67. The automatic urine collecting apparatus of claim 36, wherein the urine storage tank 21a is mounted to the housing 20 detachable forward and backward.

68. The automatic urine collecting apparatus of claim 36, wherein the urine storage tank 21a is mounted to the housing 20 detachable laterally.

69. The automatic urine collecting apparatus of claim 36, wherein the urine storage tank 21a is mounted to the housing 20 as cover rotation type.

70. The automatic urine collecting apparatus of claim 36, wherein the urine storage tank 21a is mounted to the housing 20 detachable upward and downward.

71. The automatic urine collecting apparatus of claim 1, wherein a rechargeable battery is included within the housing 20.
